# FASCICULE DE BREVET EUROPEEN

(11) **EP 0 701 553 B1**
(45) Date de publication et mention de la délivrance du brevet: **01.10.1997**
(21) Numéro de dépôt: 94917066.6
(22) Date de dépôt: 31.05.1994
(51) Int. Cl.: C07D 231/16, C07D 413/06, C07D 403/14, A01N 43/56, C07D 405/14, C07D 405/06, C07D 405/12, C07D 409/14

(54) **ARYLPYRAZOLES FONGICIDES**
ARYLPYRAZOLE ALS FUNGIZIDE
ARYLPYRAZOLE FUNGICIDES

(30) Priorité: 03.06.1993 FR 9306878
(43) Date de publication de la demande: 20.03.1996
(73) Titulaire: RHONE-POULENC AGROCHIMIE, 69009 Lyon (FR)
(72) Inventeur: PEIGNIER, Raymond, F-69300 Caluire (FR); CANTEGRIL, Richard, F-69009 Lyon (FR)
(86) Numéro de dépôt international: FR9400628
(87) Numéro de publication internationale: WO9429276

(56) Documents cités:
- EP-A- 0 433 899
- EP-A- 0 538 156
- DE-A- 3 620 825
- CHEMICAL ABSTRACTS, vol. 119, no. 20, 15 Novembre 1993, Columbus, Ohio, US; abstract no. 213909r, 'Method for processing color photographic material.' page 759 ;
- JOURNAL OF ORGANIC CHEMISTRY. vol. 57, no. 7 , 1992 , EASTON US pages 2127 - 2134 J. BECHER ET AL. 'Azide ring-opening-ring-closure reactions and tele-substitutions in vicinal azidopyrazole-, pyrrole- and indolecarboxaldehydes.'

## Description

La présente invention concerne de nouveaux dérivés de la famille des 3-arylpyrazoles, leurs procédés de préparation, les compositions les contenant et leur utilisation pour la protection des plantes contre les maladies fongiques.

On connaît d'après la demande EP 0 538 156 des 3-phénylpyrazoles fongicides avec un noyau phényle substitué en position 1 et/ou 2 .
L'invention a plus spécialement pour objet des dérivés 3-arylpyrazoles, leurs sels, leurs dérivés N-oxydes, leurs complexes métalliques et métalloïdiques de formules I et I bis: 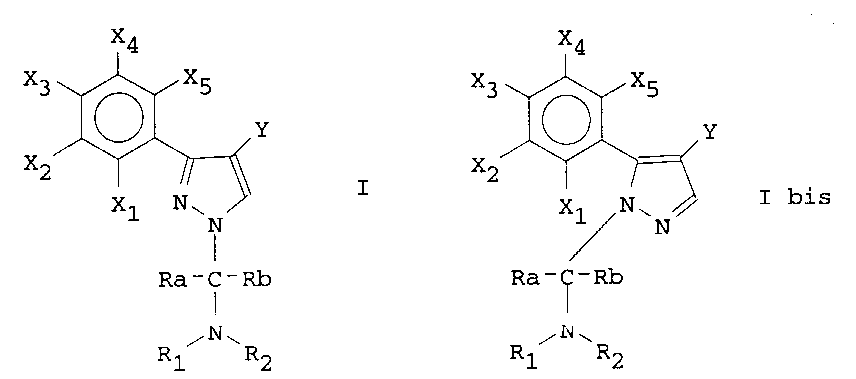 dans laquelle :
X₁, X₂, X₃, X₄ et X₅, identiques ou différents, sont:
- un atome d'hydrogène ou d'halogène, un groupe hydroxy, mercapto, cyano, thiocyanato, nitro, nitroso, amino éventuellement substitué par un ou deux alkyles ou phényles ou acyle, les imines, énamines , amidines et guanidines dérivées de ce groupe amino;
- un radical alkyle, hydroxyalkyle, alkoxyalkyle, alkylthioalkyle, alkylsulfinylalkyl, alkylsulfonylalkyl, benzyle, alkényle, alkynyle, cyanoalkyle, alkoxy, alkenyloxy, formyle, acétyle, alkyl- ou alkoxy(thio)-carbonyle, mono ou dialkylamino(thio)carbonyle, iminocarbonyl, mono ou diarylamino(thio)carbonyle, carboxyle, carboxylate, carbamoyle ou benzoyle,
- un radical phényle, phénoxy, phénylthio,
- un radical alkyle- ou alkoxy- ou mono ou dialkylamino- ou phényl-sulfényl ou sulfinyle ou sulfonyle,
- un groupe phosphoryle, substitué par deux groupes choisis dans le groupe comprenant alkyle, alkoxy, alkylthio, dialkylamino, benzyloxy, phényloxy et phényl,
- un groupe trialkyl- ou alkylphényle -silyle
étant entendu que dans toutes les significations hydrocarbonées ci-dessus, la partie alkyle de ces groupes peut comprendre de 1 à 4 atomes de carbone et être éventuellement halogénée et que phényle désigne le noyau phényle éventuellement substitué.

Deux des X₁, X₂, X₃, X₄ et X₅ adjacents peuvent également former un cycle carboné comprenant au total de 5 à 7 chainons, qui peut comporter un ou plusieurs atomes ou groupes suivants: O, S, N, C=O, C=S, SO, SO₂, CH=CH. Les carbones de ce pont peuvent ou non être substitués par au moins un atome d'halogène et/ ou au moins un groupe hydroxy, alkoxy, alkylthio, mono ou dialkylamino, alkylsulfinyle ou -sulfonyle, la partie alcoyle étant telle que définie ci-dessus,
sous réserve qu'au moins un des groupes X soit différent d'hydrogène;
Y est:
- un atome d'hydrogène ou d'halogène,
- un groupe hydroxy, mercapto, et leurs dérivés formylés, alkyl- ou alcoxy- ou amino (thio)acylés, le groupement amino pouvant être éventuellement substitué.
- un groupe nitro, cyano, thiocyanato, azido
   - un groupe alkyle, alkényle, alkynyle, alkoxy ou alkylthio, chacun de ces groupes étant éventuellement halogéné,
   - un radical acyle ou thio acyle qui peut être: un radical formyle, un groupe alkyl- ou alkényl-carbonyle ou -thiocarbonyle, le radical alkyle ou alkényle pouvant être linéaire ou ramifié
   - un groupe alkoxy- ou alkylthio- ou amino- ou monoalkylamino- ou dialkylamino- ou phénylamino- ou alkylphenylamino(thio)carbonyle
- un groupe carboxy ou ses sels,
- un phénoxy éventuellement substitué,
   - un amino substitué ou non par un ou deux alkyles ou phényles,
   - un groupe alkylsulfinyle ou alkylsulfonyle, la partie alkyle étant telle que définie ci-dessus,

Y et X₅ peuvent également former un cycle carboné comprenant au total de 5 à 7 chainons, qui peut comporter un ou plusieurs atomes ou groupes suivants: O, S, N, C=O, C=S, SO, SO₂, CH=CH. Les carbones de ce pont pouvant ou non être substitués par au moins un atome d'halogène et/ ou au moins un groupe hydroxy, alkoxy, alkylthio, mono ou dialkylamino, alkylsulfinyle ou -sulfonyle, la partie alcoyle étant telle que définie ci-dessus;
Ra est
- un atome d'hydrogène,
- un radical R pouvant être:
   - alkyl de 1 à 6 C(éventuellement substitué par GR7)
   - cycloalkyl(3 à7 C), cycloalkyl(3 à7 C)alkyl(1 à 4C) (éventuellement substitué par GR3(défini ci-après))
   - phényl, un hétérocycle Het(défini ci-après), phénylalkyl(1 à 4C), Het alkyl (1 à 4C), éventuellement substitué par 1 à 8 atomes d'halogène et GR4(défini ci-après)),
- un groupe C(V)-V'R₀ ou C(V)NR₀R'₀, dans lesquels:
   - V et V', identiques ou différents sont un atome d'oxygène ou de soufre et
   - R₀ et R'₀ identiques ou différents peuvent être un atome d'hydrogène, les radicaux R ou R', éventuellement substitués par un groupe α, qui peut être un groupe GR₁ (défini ci-après), NZ₁Z₂, SO₂NZ₁Z₂, CVZ₁Z₂, dans lesquels V est comme défini plus haut et Z₁,Z₂ sont un atome d'hydrogène ou le radical R, qui peut en outre être éventuellement substitué par GR2(défini ci-après),
Rb est un atome d'hydrogène ou peut former avec Ra, un cycle hydrocarboné de 4 à 6 C,
R₁ est:
- 1) R₀, dans lequel Z₁, Z₂ peuvent en outre former, avec l'atome d'azote auquel ils sont rattachés, un radical Het (défini ci-après), éventuellement substitué par GR3(défini ci-après); ou un radical R' pouvant être: alkényl, alkynyl, phénylalkényl, phénylalkynyl, Hetalkényl, Hetalkynyl, la partie alkényl ou alkynyl de ces radicaux ayant de 2 à 6C,
   Lorsque R1 est un groupe R0, et plus précisément lorsque R0 est Hetalkyl (1 à 4 C), il peut être plus spécifiquement un groupe de formule IIa ou IIb, 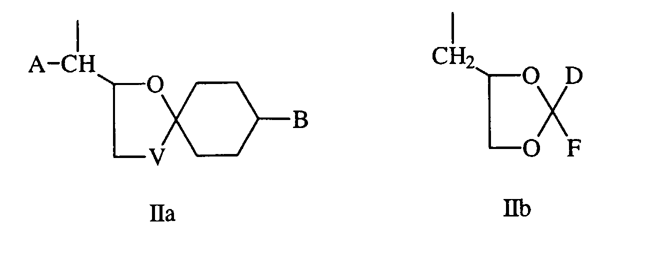 dans lesquels,
   V a la même définition que précédemment,
   A est un atome d' hydrogène ou un groupe méthyle,
   Sous réserve que V est un atome d' oxygène lorsque A est un groupe méthyle .
   B est un groupe: alkyl (1 à 14 C), cycloalkyl (3 à 7 C), cycloalkylalkyl (3 à 7 C; 1 à 8 C pour la partie alkyl), aryl (6 à 10 C), aralkyl ( 6 à 10 C; 1 à 8 C pour la partie alkyl).
   D est un groupe:
      Alkyl (1 à 14 C),
      Halogénoalkyl (1 à 14 C pour la partie alkyl; 1 à 6 atomes d' halogène),
      Cyanalkyl (1 à 4 C pour la partie alkyl),
      Aralkyl, Aryloxy(thio)alkyl, Arylsulfi(o)nylalkyl, Aralkyloxy(thio)alkyl (6 à 10 C pour la partie Aryl; 1 à 8 C pour la partie alkyl; la partie Aryl éventuellement substituée par un ou plusieurs substituant(s) choisi(s) parmi le groupe GR4).
   F, identique à ou différent de D, et pouvant avoir les mêmes significations, peut être en outre un atome d' hydrogène.
- 2) R₃, qui peut être:
   - un atome d'hydrogène,
      un groupe SO₂W₁, dans lequel W₁ est le groupe R ou R'
   - un groupe C(V)W₂, dans lequel W₂ est un groupe R₀,
   VR₀, NR₀R'₀, dans lequel R₀ est tel que défini plus haut,
   - un groupe P(V)W₃, W'₃ dans lequel W₃, W'₃, identiques ou différents peuvent être un groupe R, VR ou R', W₃, W'₃, pouvant en outre former, avec l'atome de phosphore auquel ils sont rattachés, un radical Het, éventuellement substitué par GR4(défini ci-après),
   - un groupe CW₄=N-W'₄, dans lequel W₄ et W'₄, identiques ou différents peuvent être un groupe nitro, cyano, R₀, VR₀, C(V)V'R₀, et C(V)NR₀R'₀, dans lesquel R₀ et R'₀ sont tels que définis plus haut, R₀ pouvant en outre, pour C(V)V'R₀, être un atome de métal alcalin ou alcalinoterreux; W₄ et W'₄, pouvant en outre former, avec l'atome d'azote auquel ils sont rattachés, un radical Het, éventuellement substitué par GR3 (défini ci-après),
- 3) un groupe G1-T1-Rc, dans lequel :
   - G1 est un groupe SO2,C(V), P(V)W3 et C=N-W4,
   - T1 est V, CH ou Cα,
      G1 et T1 pouvant en outre former un groupe N=CW4, les symboles V, W3, W4 et α étant tels que définis ci-dessus;
   - Rc est un alkyle de 1 à 6 C;
- 4) un groupe C(O)-R₀, dans lesquel R₀ est tel que défini plus haut,
- 5) un groupe NTT', dans lequel:
   - T est R₀, G1-T1-Rc ou N=CW3W4
      et T' est R₀ ou R₃, Rc , G2-T2 Rc ou G1-T1 Rc;
      R₀, R₃, Rc, G1, G2, T1 et T2 étant tels que définis ci-dessus;
      G2 et T2 étant respectivement identiques à ou différents de G1 et T1; et T et T' ne pouvant être ensemble un atome d'hydrogène et pouvant former un groupe morpholino ou pipéridino;
- 6) un groupe C(CRaRbNR₄)ₙCRaRbR₄, dans lequel n est égal à zéro ou 1, Ra, Rb sont tels que définis plus haut, R₄ est un radical de formule: 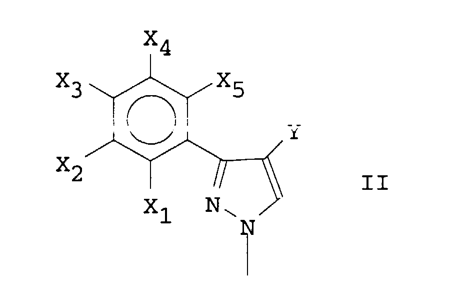 et R₅ est R₁ ou CRaRbR₂,
- 7) un groupe C(L)NT'T", dans lequel L est V, T" est R₀, G1-T1-Rc ou CRaRbR₄, et dans lequel Ra, Rb, R₄ , V et T' sont tels que définis ci-dessus,
T' et T" ne pouvant être à la fois un atome d'hydrogène et pouvant former un groupe morpholino ou pipéridino;

R₂, identique à ou différent de R₁, et pouvant avoir les mêmes significations, c'est à dire pouvant être R₀, OR₀, R₃, Rc, G1-T1-Rc, G2-T2-Rc, et CRaRbR₄, sous réserve qu'ils ne soient pas simultanément un atome d'hydrogène,
R₁ et R₂ pouvant en outre former, avec l'atome d'azote auquel ils sont rattachés:
- un radical Het azoté, éventuellement substitué par GR3, de préférence un morpholino ou pipéridino; ou pyrazolidino;
- un groupe N=CH-W5, dans lequel W5 est R, Rb étant un atome d'hydrogène
et les sels(halogénures,carboxylates et sulfates) d'ammonium substitué et d'imidinium substitué de ces composés.

Lorsque R1 est un groupe de formule IIa ou IIb, alors R2 est un atome d' hydrogène ou un groupe R défini comme précédemment.
étant donné que dans ce qui précède, sauf indication spéciale:
"alkyl" désigne un alkyl contenant de 1 à 4 atomes de carbone,
"cycloalkyl" désigne un cycloalkyl contenant de 1 à 4 atomes de carbone,
"alkényl" ou "alkynyl" désigne un alkényl ou alkynyl de 2 à 5 atomes de carbone,
"Het" est un radical hétérocyclique,mono ou bicyclique, contenant de 5 à 10 atomes de carbone, dont 1 à 4 sont N, O, S ou P.

GR1 est:
- un atome d'halogène ou un groupe nitro ou cyano,
- un groupe R, R', OR, RS(O)m, avec m égal à 1 à 3, RC(V)V' ou R'C(V)V'

GR2 est:
- un atome d'halogène ou un groupe nitro ou cyano,
- un groupe R, R', OR, RS(O)m, avec m égal à 1 ou 3, RC(V)V' ou R'C(V)V',
- un groupe mono ou dialkyl amino, mono ou dialkyl amino(thio) carbonyl, mono ou dialkyl aminosulfonyl.

GR3 est:
- un atome d'halogène ou un groupe nitro ou cyano,
- un groupe R, OR, RS(O)m, avec m égal à 1 ou 3, RC(V)V' ou
- un groupe mono ou dialkyl amino, mono ou dialkyl amino(thio)carbonyl, mono ou dialkyl aminosulfonyl.

GR4 est:
- un atome d'halogène ou un groupe nitro ou cyano,
- alkyl, alkoxy, alkylsulfényl, alkylsulfonyl, alkyl(thio)carbonyl, alkoxy(thio)carbonyl,
- un groupe mono ou dialkyl amino, mono ou dialkyl amino(thio) carbonyl, mono ou dialkyl aminosulfonyl.

GR5 est :
- un atome d'halogène ou un groupe nitro ou cyano,
- un groupe R, R', OR, RS(O)3, RC(V)V' ou R'C(V)V',
- un groupe mono ou dialkyl amino, mono ou dialkyl amino(thio) carbonyl, mono ou dialkyl aminosulfonyl.

GR6 est GR5 sauf R' et R'C(V)V'.

GR7 est GR2 sauf alkyl;
tous les radicaux de ces substituants étant définis ci-dessus.

Des composés préférés sont ceux, dans la formule desquels X1 à X5 sont choisis dans le groupe comprenant un atome d'hydrogène ou d'halogène, cyano, nitro,ou un radical alkyle de 1 à 4 atomes de carbone ou deux X voisins formant avec le phényle un benzodioxole éventuellement halogéné.

D'autres composés préférés sont ceux, dans la formule desquels Y est un atome d'halogène ou un groupe cyano.

D'autres composés préférés sont ceux, dans la formule desquels Ra et Rb, identiques ou différents, sont un atome d'hydrogène ou un alkyle de 1 à 4 atomes de carbone.

D'autres composés préférés sont ceux, dans la formule desquels R₁, R₂, identiques ou différents, sont un radical R.

D'autres composés préférés sont ceux, dans la formule desquels R₁, R₂, identiques ou différents, sont un alkyle de 1 à 4 atomes de carbone.

D'autres composés préférés sont ceux, dans la formule desquels R₁, R₂, forment, avec l'atome d'azote qui les porte un groupe morpholino, pipéridino, pyrolidino, imidazolyl, triazolyl, pyrazolyl ou benzoxazinyl.

Les composés de formule I selon l'invention peuvent être préparés selon différents procédés.

Selon un premier procédé de préparation de composés de formule I, dans laquelle R₁, R₂, identiques ou différents sont choisis dans R₀, on fait agir un aldéhyde RaCH(O) ou d'une cétone RaRbC(O), en présence d'une amine R₁R₂NH, sur un phényl pyrazole de formule II-H: 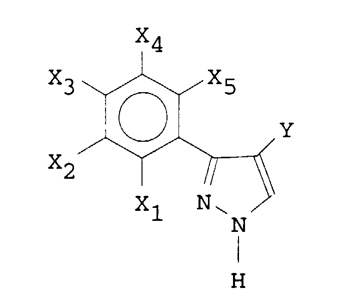 dans laquelle X₁, X₂, X₃, X₄ et X₅ et Y ont les mêmes significations que dans la formule I, à une température comprise entre l'ambiante et le point de reflux, en milieu solvant, éventuellement en présence d'une quantité catalytique d'acide fort.

Comme exemple milieu solvant on peut utiliser d'une manière générale un solvant, ou un mélange de solvants, aromatique choisi dans le groupe comprenant le toluène et le xylène, ou un solvant ou un mélange de solvants, aprotique choisi dans le groupe comprenant diéthyléther, tetrahydrofurane, acétone, acétonitrile ou encore un solvant, ou un mélange de solvants, protique polaire choisi dans le groupe comprenant l'eau, le méthanol, l'éthanol, l'isopropanol et l'acide acétique.

Comme d'exemple d'acide fort on peut utiliser l'acide acétique et l'acide paratoluènesulfonique.

Selon un second procédé pour la préparation de sels d'ammonium substitué ou d'imidinium substitué des composés de formule I, dans laquelle on fait réagir un pyrazole de formule R₄CRaRbU, dans laquelle R₄, Ra et Rb sont définis comme ci-dessus et U est un atome d'halogène ou un groupe libérable tel que mésyle ou tosyle, sur une quantité stoechiométrique d'une amine tertiaire RNR₁R₂, aliphatique ou hétérocyclique à au moins un azote salifiable, à une température comprise entre l'ambiante et le point de reflux, en milieu solvant, ou un mélange de solvants, ou un solvant ou un mélange de solvants, aprotique choisi dans le groupe comprenant l'acétone, l'acétonitrile ou encore un solvant, ou un mélange de solvants, protique polaire choisi dans le groupe comprenant le méthanol, l'éthanol, ou encore polaire tel que le N,N-diméthylformamide et la N-méthyl pyrrolidone.

Selon un troisième procédé, pour la préparation de composés de formule I, dans laquelle R₁ et R₂ sont un groupe CRaRbR₄, c'est à dire de formule R₄CRaRbNCRaRbR₄, caractérisé en ce qu'on fait réagir un hydroxyméthyl -pyrazole de formule R₄CRaRbOH sur un pyrazole dimère de formule R₄CRaRbNHCRaRbR₄ ou sur du formamide, en milieu solvant aromatique ou protique polaire, à une température comprise entre l'ambiante et le point de reflux, en milieu solvant, éventuellement en présence d'une quantité catalytique d'acide fort.

Selon un quatrième procédé, pour la préparation de composés de formule I, dans laquelle R1 et R2 forment ensemble avec l'atome d'azote qui les porte, un groupe N=CH-W5, on fait agir un aldéhyde RaCH(O), en présence d' ammoniac alcoolique, sur un phényl pyrazole de formule II défini ci-dessus, à une température comprise entre l'ambiante et le point de reflux, en milieu solvant protique polaire, éventuellement en présence d'une quantité catalytique d'acide fort.

Selon un cinquième procédé pour la préparation des composés de formule I, dans lequel on fait réagir un pyrazole de formule R4CRaRbU dans laquelle R₄, Ra et Rb, U sont définis comme ci-dessus, sur 1,0 à 1,5 équivalent d' une amine de formule R1R2NH,
- en présence de 1,0 à 1,5 équivalent d'une base choisie parmi K2CO3, Na₂CO₃, NaHCO₃, Et₃N, N,N-Diméthyl Aniline, Pyridine, 4-Diméthylamino Pyridine (DMAP), Diazabicyclooctane (DABCO), Diazabicyclononène (DBN), Diazabicycloundécène (DBU).
- en présénce ou absence d' un solvant tel que toluène, xylènes, chlorobenzènes, cyclohexane, N,N-Diméthyl Formamide (DMF), N,N-Diméthyl Acétamide (DMA), N-Méthyl Pyrrolidone (NMP), Diméthylsulfoxyde (DMSO), EtOH, i-PrOH, n-BuOH.
- à une température comprise entre 20 °C et 180 °C.

Les amines de formule IVa ou IVb peuvent être préparées selon les méthodes ou une adaptation des méthodes décrites dans EP 281 842 et / ou DE 3 828 545 et / ou DE 3 305 769, 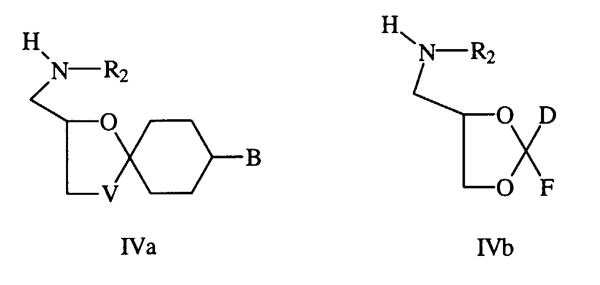 par réaction d' un hétérocycle de formule Va ou Vb, 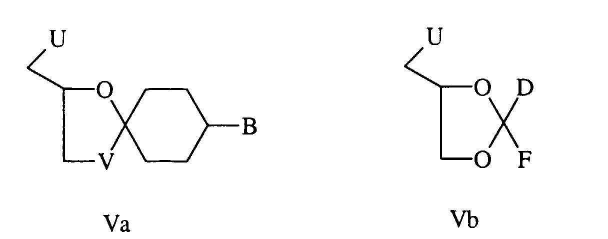 dans lesquels,
U, V, B, D, F ont la même définition que précédemment,
avec 1,0 à 2,5 équivalent(s) d' une amine de formule R2NH2,
   - en présence de 1,0 à 2,5 équivalent(s) d' une base choisie parmi K2CO3, Na₂CO₃, NaHCO₃, Et₃N, N,N-Diméthyl Aniline, Pyridine, 4-Diméthylamino Pyridine (DMAP), Diazabicyclooctane (DABCO), Diazabicyclononène (DBN), Diazabicyclo-undécène (DBU).
   - en présence ou absence d' un solvant tel que toluène, xylènes, chlorobenzènes, cyclohexane, N,N-Diméthyl Formamide (DMF), N,N-Diméthyl Acétamide (DMA), N-Méthyl Pyrrolidone (NMP), Diméthylsulfoxyde (DMSO), EtOH, i-PrOH, n-BuOH.
   - à une température comprise entre 20 °C et 180 °C.

Les hétérocydcs de formule Va peuvent être obtenus par analogie avec:
J. Org. Chem. 1973, 38, 834-35.
Tet. Letters 1982, 23, 47-50.
Liebigs Ann. Chem. 1984, 1298-1301.
J. Org. Chem. 1988, 51, 1894-97.
Z. Naturforsch. B. Anorg. Chem. Org. Chem. 1985, 4013, 393-97.
par réaction d' une cyclohexanone de formule VIa, avec 1,0 à 2,0 équivalent(s) d' un alcool de formule VIIa, 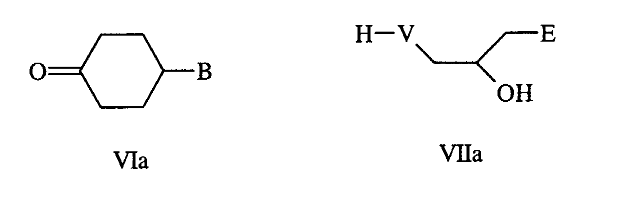 dans lequel,
V a la même définition que précédemment,
E est un atome d' halogène ou un groupe hydroxy,
dans un solvant tel que toluène, xylènes, chlorobenzènes, cyclohexane, en présence d' une quantité catalytique d' acide p-toluène sulfonique (APTS),
à un température comprise entre 40 °C et 150 °C.

Les hétérocycles de formule Vb peuvent être préparés par analogie avec:
Rec. Trav. Chim. Pays-Bas 1972, 91, 989-1001; Farm. Ed. Sci. 1974, 29, 167-74; J. Med. Chem. 1963, 6 (3), 325-28; par réaction d' une cétone ou aldéhyde de formule VIb, avec 1,0 à 2,0 équivalent(s) d' un alcool de formule VIIb, 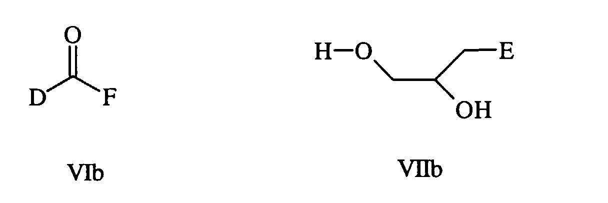 dans lesquels,
D, F, E ont la même définition que ci-dessus,
dans un solvant tel que toluène, xylènes, chlorobenzènes, cyclohexane,
en présence d' une quantité catalytique d' acide p-toluène sulfonique (APTS), à un température comprise entre 40 °C et 150 °C.

Les cyclohexanones de formule VIa peuvent être préparées par analogie avec:
Tet. Letters 1987, 28, 2347-50; Tet. Letters 1986, 27, 2875-78;Tet. Letters 1979, 19, 3209-12; J. Amer. Chem. Soc. 1987, 109, 6887-89; J. Amer. Chem. Soc. 1973, 95, 3646-51;
J. Amer. Chem. Soc.1972, 94, 7599-7600; Bull. Chem. Soc. Jap. 1987, 60, 1721-26,
Synth. Commun. 1985, 15, 759-64; Synth. Commun. 1982, 12, 267-77; J. Org. Chem. 1973, 38, 1775-76; US 4 251 398; US 3 960 961; EP 0 002 136; DE 2 636 684; DE 2 509 183;
FR 2 231 650.

Les amines de formule VIII peuvent être préparées selon les méthodes ou une adaptation des méthodes décrites dans EP 355 597 par réaction d' un hétérocycle de formule IX, 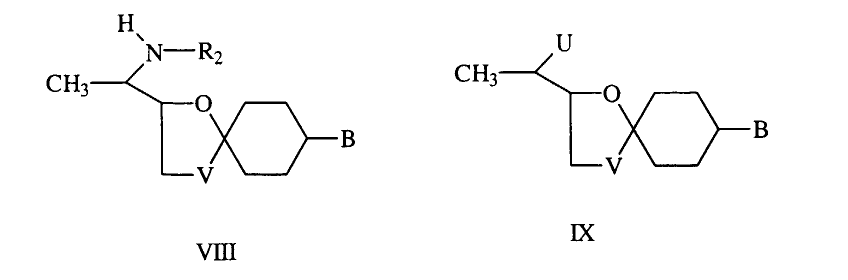
dans lesquels U et B ont la même définition que ci-dessus,
avec 1,0 à 2,5 équivalent(s) d' une amine de formule R₂NH₂,
dans les mêmes conditions précédemment décrites pour la préparation de Va et Vb,

Les hétérocycles de formule IX peuvent être obtenus par réaction d' une cyclohexanone de formule VIa, avec 1,0 à 2,0 équivalent(s) d'un alcool de formule X, ou un oxiranne de formule XI, 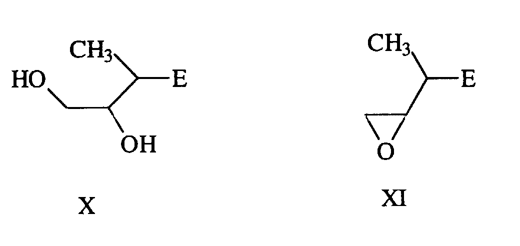
dans lesquels E a la même définition que ci-dessus,
dans un solvant tel que toluène, xylènes, chlorobenzènes, cyclohexane,
en présence d' une quantité catalytique d' acide p-toluène sulfonique (APTS), à un température comprise entre 40 °C et 150 °C.

Les alcools de formule X peuvent être préparés par analogie selon les procédés décrits dans : EP 200 267; DE 2 937 840; US 4 035 178; Tetrahedron 1971, 27, 3197-3205; Tetrahedron 1979, 35, 2583-89.

Les oxirannes de formule XI peuvent être préparés selon les procédés décrits dans: J. Amer. Chem. Soc. 1974, 96, 5254-55; Tet. Letters 1977, 17 , 4397-400; Tet. Letters 1979, 19, 4733-36; Tet. Letters 1980, 20, 4843-46.

L'invention a encore pour objet des composés de formules
R4CRaRbNHCRaRbR4, R4CRaRbOH, R4CRaNHCHO et R4CRaCN utilisables comme intermédiaires pour la préparation des composés de formule I selon l'un des procédés décrits ci-dessus

L'invention a encore pour objet des compositions pour la protection des plantes contre les maladies fongiques, caractérisées en ce qu'elles contiennent comme matière active au moins un composé de formule I.

Les exemples suivants sont donnés à titre indicatif pour illustrer la préparation et l'activité fongicide des dérivés selon l'invention. La structure de ces derniers a été confirmée par analyse RMN.

### Exemple 1:

### 1-((2,6-Diméthylmorpholin-4-yl)méthyl), 4-chloro, 3-(2-nitro, 3-chloro)phényl pyrazole.

Une goutte d'acide acétique est additionnée à une solution de 1,80 g (0,007 mole) de 4-chloro- 3-(2-nitro- 3-chloro)phényl pyrazole et 0,55 g (0,00735 mole) de formaldéhyde aqueux (37%) dans 30 ml d'éthanol. Une solution de 0,85 g (0,00735 mole) de 2,6-diméthyl morpholine dans 10 ml d'éthanol est additionnée goutte à goutte à température ambiante. L'ensemble est agité 5 heures à la même température puis concentré à sec sous pression réduite. Le solide résiduel est repris avec 50 ml d'eau, extrait avec 50 ml d'éther éthylique. La phase organique est séchée sur MgSO₄, concentrée sous pression réduite. Nous obtenons 2,5 g (0,0065 mole) de 1-((2,6-diméthylmorpholin-4-yl)méthyl), 4-chloro, 3-(2-nitro-3-chloro)phényl pyrazole fondant à 169°C.

Sont préparés comme à l'exemple 1 les composés de formule III: 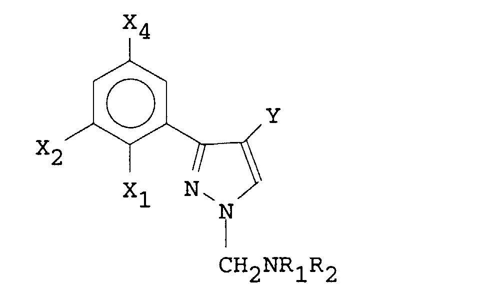 rassemblés dans le tableau suivant:

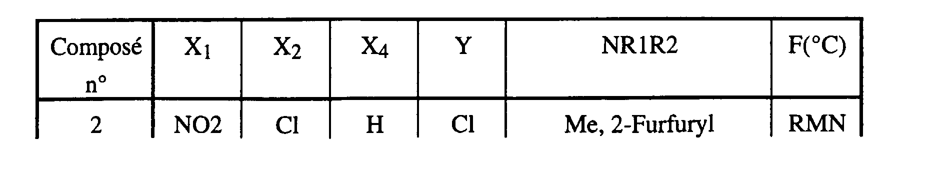

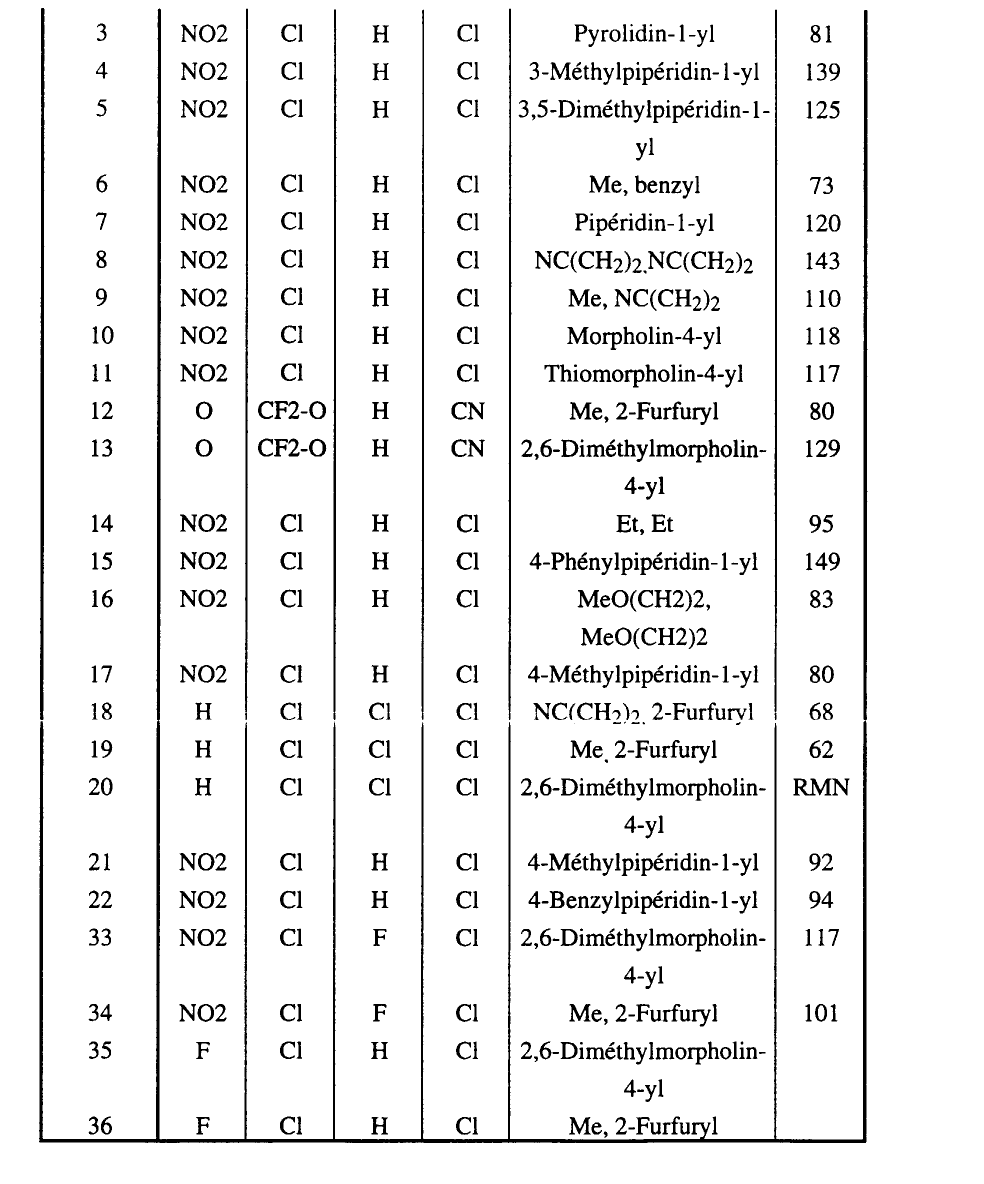

### Exemple 2 :

### 1-(Imidazol-1-yl)méthyl, 4-chloro, 3-(3,5-dichloro)phényl pyrazole(composé 23).

0,15 ml de 1,8-diazabicyclo(5.4.0)undécèn-7-ène est additionné, à température ambiante, à une solution de 2,55 g (0,01 mole) de 4-chloro, 3-(3,5-dichloro)phényl pyrazole et 0,90 g (0,03 mole) de paraformaldéhyde dans 70 ml de THF. Le mélange réactionnel est agité 4 heures à température ambiante. Une solution de 4,75 g (0,015 mole) de chlorure de thionyle dans 20 ml de THF est coulée au goutte à goutte à 0°C, et l'agitation poursuivie 4 heures à température ambiante. Le milieu réactionnel est concentré à sec. Le résidu est repris avec 15 ml d'heptane puis séché. Nous obtenons 2,15 g (0,0073 mole) de 1-chlorométhyl, 4-chloro, 3-(3,5-dichlorophényl)pyrazole fondant à 88°C.

1,1 g (0,0074 mole) d'iodure de sodium est additionné à une solution de 2,15 g (0,0073 mole) de 1-chlorométhyl, 4-chloro, 3-(3,5-dichloro)phényl pyrazole dans 40 ml d'acétone anhydre. L'ensemble est agité 4 heures à température ambiante. Le chlorure de sodium est éliminé par filtration et le milieu réactionnel dilué avec 30 ml de DMF anhydre. Après addition de 0,51 g (0,0075 mole) d'imidazole et 1,05 g (0,0076 mole) de K₂CO₃, l'agitation est poursuivie 2 heures à 60°C. Le milieu réactionnel est versé dans 250 ml d'eau glacée. Le précipité est récupéré par filtration, lavé avec 20 ml d'eau et 20 ml d'heptane, séché sous pression réduite. Nous obtenons 1,95 g (0,006 mole) de 1-(imidazol-1-yl)méthyl, 4-chloro, 3-(3,5-dichloro)phényl pyrazole.fondant à 157°C.

Sont préparés comme à l'exemple 2 les composés de formule III, rassemblés dans le tableau suivant:

| Composé n° | X₁ | X₂ | X₄ | Y | NR1R2 | F(°C) |
|---|---|---|---|---|---|---|
| 24 | H | Cl | Cl | Cl | [1,2,4]Triazol-1-yl | 177 |
| 25 | H | Cl | Cl | Cl | Pyrazol-1-yl | 205 |

### Exemple 3 :

### 1-(1,4-Dihydro-2H-3,1-benzoxazin-1-yl)méthyl, 4-chloro, 3-(3,5-dichloro)phényl pyrazole(composé 26).

Une goutte d'acide acétique est additionnée à une solution de 2,55 g (0,01 mole) de 4-chloro, 3-(3,5-dichloro)phényl pyrazole et 2 ml (0,025 mole) de formaldéhyde aqueux (37%) dans 30 ml d'éthanol. Une solution de 1,25 g (0,01 mole) d'alcool o-(amino) benzylique dans 10 ml d'éthanol est coulée au goutte à goutte et à température ambiante. L'ensemble est agité 5 heures à la même température puis concentré à sec sous pression réduite. Le solide résiduel est repris avec 50 ml d'eau, extrait avec 50 ml d'éther éthylique. La phase organique est séchée sur MgSO₄, concentrée sous pression réduite. Nous obtenons 3,60 g (0,0091 mole) de 1-(1,4-dihydro-2H-3,1-benzoxazin-1-yl)méthyl, 4-chloro, 3-(3,5-dichloro)phényl pyrazole fondant à 143°C.

### Exemple 4 :

### Chlorure de (4-chloro, 3-(3,5-dichloro)phényl pyrazol-1-yl) triéthylammonium méthane(composé 27).

1,1 g (0,0074 mole) d'iodure de sodium est additionné à une solution de 2,15 g (0,0073 mole) de 1-chlorométhyl, 4-chloro, 3-(3,5-dichloro)phényl pyrazole (préparé comme à l'exemple 2) dans 40 ml d'acétone anhydre. L'ensemble est agité 4 heures à température ambiante. Le chlorure de sodium est éliminé par filtration. 0,76 g (0,0075 mole) de triéthyl amine est additionné au filtrat et le milieu réactionnel agité 12 heures à température ambiante. Le précipité est récupéré par filtration, lavé avec 20 ml d'heptane,séché sous pression réduite. Nous obtenons 2,55 g (0,00525 mole) de chlorure de (4-chloro, 3-(3,5-dichloro)phényl pyrazol-1-yl)méthyl triéthylammonium fondant à 175°C.

### Exemple 5 :

### 1,1-Bis((4-chloro, 3-(3,5-dichloro)phényl pyrazol-1-yl)méthyl), 4-(diméthylamino) aniline(composé 28).

Quelques cristaux d'acide p-toluène sulfonique sont additionnés à une solution de 2,55 g (0,01 mole) de 4-chloro, 3-(3,5-dichloro)phényl pyrazole et 2,4 ml (0,03 mole) de formaldéhyde aqueux (37%) dans 30 ml de toluène. Une solution de 0.70 g (0.005 mole) de 4-(diméthylamino) aniline dans 10 ml de toluène est coulée au goutte à goutte et à température ambiante. L'ensemble est agité 5 heures à la température du reflux puis concentré à sec sous pression réduite. Le solide résiduel est repris avec 50 ml d'eau, 50 ml d'heptane, puis séché sous pression réduite. Nous obtenons 2,80 g (0,0043 mole) de 1,1-Bis((4-chloro, 3-(3,5-dichloro)phényl pyrazol-1-yl)méthyl), 4-(diméthylamino) aniline. fondant à 181°C.

Sont préparés comme à l'exemple 5 les composés de formule IV, 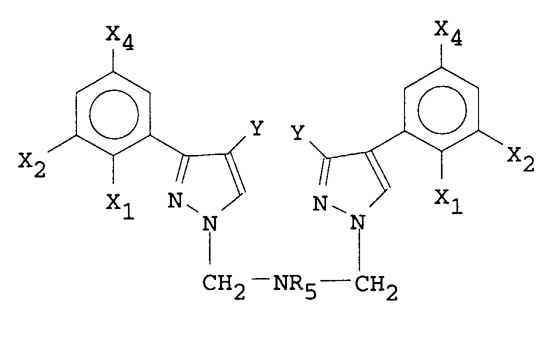 rassemblés dans le tableau suivant:

| Composé n° | X₁ | X₂ | X₄ | Y | R5 | F(°C) |
|---|---|---|---|---|---|---|
| 29 | H | Cl | Cl | Cl | tetrahydrofury-2-yl | 130 |
| 30 | H | Cl | Cl | Cl | OH | 183 |
| 31 | H | Cl | Cl | Cl | Me2N(CH2)2 | 95 |

### Exemple 6 :

### 1,3-Bis((4-chloro, 3-(3,5-dichloro)phényl pyrazol-1-yl)méthyl), 2,3-dihydrobenzimidazole(composé 32).

Une goutte d'acide acétique est additionnée à une solution de 2,55 g (0,01 mole) de 4-chloro, 3-(3,5-dichloro)phényl pyrazole et 2,4 ml (0,03 mole) de formaldéhyde aqueux (37%) dans 30 ml d'éthanol. Une solution de 0,55 g (0,005 mole) de 1,2-phénylène diamine dans 10 ml d'éthanol est coulée au goutte à goutte et à température ambiante. L'ensemble est agité 5 heures à 60°C. Le précipité est récupéré par filtration, lavé à l'heptane puis séché sous pression réduite. Nous obtenons 0,87 g (0,00135 mole) de 1,3 Bis((4-chloro, 3-(3,5-dichloro)phényl pyrazol-1-yl)méthyl), 2,3-dihydrobenzimidazole fondant à 170°C.

De la même manière on a obtenu le 1,3-Bis (4-chloro, 3-(3,5-dichloro)phényl pyrazol-1-yl)méthyl), 2,3-dihydroimidazole(composé 33) de point de fusion 139°C.

### Exemple 7: 8-t-butyl-2-chlorométhyl-1,4-dioxaspiro-(4,5)-décane .

97,0 g (0,597 mole) de 4-t-butylcyclohexanone et 134,7 g (1,184 mole) de 3-chloropropane-1,2-diol sont mis en solution dans 1100 ml de toluène en présence de 11,5 g (0,060 mole) d' APTS. L' ensemble est porté au reflux pendant 6 heures en soutirant l' eau formée à l' aide d' une trappe de Dean-Stark. Le mélange réactionnel est ramené à température ambiante, lavé deux fois avec 1500 ml d' une solution aqueuse contenant 5% de NaHCO₃. La phase organique est séchée sur MgSO₄, concentrée sous vide. L' huile résiduelle est utilisée directement dans 1' étape suivante (rendement: 99%; analyse RMN).

### Exemple 8: 8-t-butyl-2-(cyclohexylaminométhyl)-1,4-dioxaspiro-(4,5)-décane.

Dans un autoclave, 6,5 g (0,0250 mole) de 8-t-butyl-2-chlorométhyl- 1,4-dioxaspiro-(4,5)-décane et 4,6 g (0,0450 mole) de cyclohexylamine sont mis en solution dans 50 ml d' éthanol à 95% en présence de 5,65 g (0,040 mole) de K₂CO₃ et 0,42 g (0,0025 mole) de KI. L' ensemble est chauffé sous agitation à 180 °C pendant 8 heures. Après retour à température ambiante, le milieu réactionnel est versé dans 500 ml d' eau, extrait avec 200 ml de CH₂Cl₂. La phase organique est séchée sur sulfate de magnésium, concentrée sous vide. L'huile brune est utilisée directement dans l' étape suivante (rendement 98%; analyse RMN).

### Exemple 9: 4-chloro-3-(2-nitro-3-chlorophényl)-1-(N-cyclohexyl-1,4-dioxaspiro(4,5)décan-8-(t-butyl)-2- méthanaminométhyl)pyrazole(composé 37)

a) On ajoute, à température ambiante, 0,40 ml de 1,8-diazabicyclo (5.4.0) undécèn-7-ène à une solution de 7,0 g (0,027 mole) de 4-chloro-3-(2-nitro-3-chlorophényl) pyrazole et 2,40 g de paraformaldéhyde dans 100 ml de THF. Le mélange réactionnel est agité pendant 4 heures à température ambiante, concentré sous pression réduite, repris avec un mélange de 150 ml d'eau et 150 ml de chlorure de méthylène. La phase organique est séchée sur sulfate de magnésium, concentrée sous vide. On obtient 7,40 g de 1-hydroxyméthyl-4-chloro-3-(2-nitro-3-chlorophényl) pyrazole fondant à 146°C(rendement: 95,5%).
b) On ajoute, goutte à goutte, à température ambiante, 1,25g (0, 0040 mole) de 8-t-butyl-2-(cyclohexylaminométhyl)-1,4-dioxaspiro-(4,5)-décane dissous dans 10 ml d' éthanol à 95% à une solution de 1,21 g (0,0038 mole) de 1-hydroxyméthyl-4-chloro-3-(2-nitro-3-chlorophényl) pyrazole dans 10 ml d'éthanol à 95%. L'ensemble est agité 12 heures à température ambiante. Le milieu réactionnel est versé dans un mélange de 70 ml d' eau et 70 ml de CH₂Cl₂. La phase organique est séchée sur sulfate de magnésium, concentrée sous vide. On obtient 2,25 g de 4-chloro-3-(2-nitro-3-chlorophényl)-1-(N-cyclohexyl-1,4-dioxaspiro(4,5)décan-8-(t-butyl)-2- méthanaminométhyl)pyrazole (rendement 99; analyse RMN).

En procédant de manière analogue on a obtenu les composés suivants de formules XII et XIII et consignés dans les tableaux suivants:

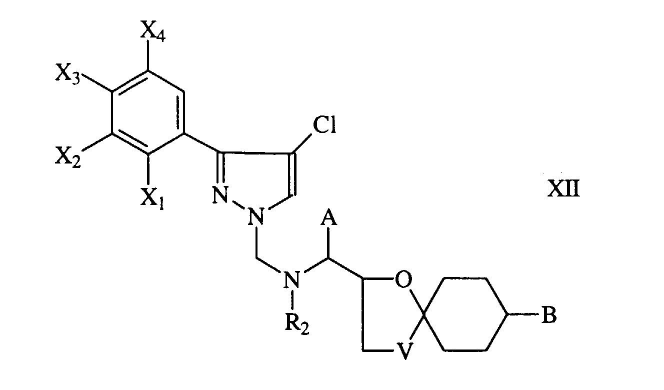

| Composé n° | X1, X2, X3, X4 | R2 | A | V | B | F°C |
|---|---|---|---|---|---|---|
| 38 | NO2, Cl, H, H | n-propyl | H | O | t-Bu | |
| 39 | NO2, Cl, H, H | i-propyl | H | O | t-Bu | |
| 40 | NO2, Cl, H, H | 2-méthylaminoéthyl | H | O | t-Bu | |
| 41 | NO2, Cl, H, H | 2-méthoxyéthyl | H | O | t-Bu | |
| 42 | NO2, Cl, H, H | cyclohexylméthyl | H | O | t-Bu | |
| 43 | NO2, Cl, H, H | benzyl | H | O | t-Bu | |
| 44 | NO2, Cl, H, H | furfuryl | H | O | t-Bu | |
| 45 | NO2, Cl, H, H | tetrahydrofurfuryl | H | O | t-Bu | |
| 46 | NO2, Cl, H, H | thièn-2-ylméthyl | H | O | t-Bu | |
| 47 | NO2, Cl, H, H | 2-morpholin-1'yl-éthyl | H | O | t-Bu | |

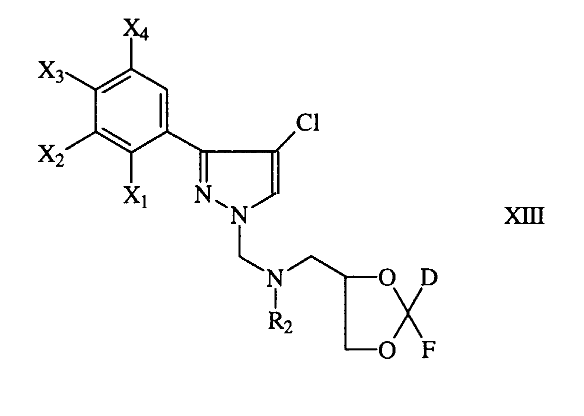

| Composé n° | X1, X2, X3, X4 | R2 | A | F | F°C |
|---|---|---|---|---|---|
| 48 | NO2, Cl, H, H | i-propyl | CH2S(4-Cl-C6H4) | t-Bu | |
| 49 | NO2, Cl, H, H | benzyl | CH2O(4-Cl-C6H4) | t-Bu | |
| 50 | NO2, Cl, H, H | furfuryl | CH3 | i-Bu | |

### EXEMPLE 10: Test in vivo Botrytis cinerea sur concombre

Une suspension aqueuse, de concentration 1 g/l, de la matière active testée est obtenue par broyage de 60 mg de celle-ci dans le mélange suivant de 5 ml d' acétone et 0,3 ml d' un agent tensioactif (TWEEN 80, oléate de dérivé polyoxyéthyléné du sorbitan) dilué à 10 % : puis le volume est ajusté à 60 ml avec de l'eau.

Cette suspension aqueuse est ensuite diluée par de l'eau pour obtenir la concentration désirée en matière active.

Des plants de concombre (variété Marketer), semés sur un substrat tourbe terre-pouzzolane 50/50 et cultivés 11 jours en serre, sont traités par pulvérisation de la suspension de matière active décrite ci-dessus.

Des plants, utilisés comme témoins, sont traités par pulvérisation d'une solution aqueuse ne contenant pas la matière active.

24 heures après traitement, l'inoculum est apporté par dépôt de gouttes d'une suspension de spores de *Botrytis cinerea,* sensibles aux benzimidazoles ou résistants aux benzimidazoles, obtenue à partir de cultures de 15 jours, mises ensuite en suspension à raison de 150 000 unités par cm3.

Après contamination, les plants sont placés en atmosphère saturée en humidité. La notation, en comparaison avec les plants témoins, est effecturée 6 jours après contamination.

Dans ces conditions, on observe à la dose de 1 g/l, une bonne protection (au moins 75%) ou totale avec les composés 1 à 11, 14 à 22, 30 et 31.

### EXEMPLE 11 : Test in vivo sur Pyriculariaoryzae responsable de la piriculariose du riz :

On prépare, par broyage fin, une suspension aqueuse de la matière active à tester ayant la composition suivante :
- matière active : 60 mg
- agent tensioactif Tween 80, oléate de dérivé polyoxyéthyléné du sorbitan) dilué à 10% dans l'eau : 0,3 ml
- on complète à 60 ml d'eau.

Cette suspension aqueuse est ensuite diluée par de l'eau pour obtenir la concentration désirée en matière active.

Du riz, semé en godets dans un mélange 50/50 de tourbe enrichie et de pouzzolane, est traité au stade 10 cm de hauteur par pulvérisation de la suspension aqueuse ci dessus.

Au bout de 24 heures, on applique sur les feuilles une suspension aqueuse de spores de *Pyricularia oryzae,* obtenue à partir d' une culture de 15 jours, mise ensuite en suspension à raison de 100 000 unités par cm3.

Les plants de riz sont placés pendant 24 heures en incubation ( 25°C, 100% d'humidité relative), puis mis en cellule d'observation, dans les mêmes conditions, pendant 5 jours.

La lecture se fait 6 jours après la contamination.

Dans ces conditions, on observe, à la dose de 1 g/l, une protection bonne (au moins 75%) ou totale avec les composés suivants: 1 à 22, 29 et 31.

### EXEMPLE 12: Test in vivoAlternaria brassicaesur radis

Une suspension aqueuse, de concentration 1 g/l, de la matière active testée est obtenue par broyage de 60 mg de celle-ci dans le mélange suivant :
- acétone : 5 ml
- agent tensioactif (TWEEN 80, oléate de dérivé polyoxyéthyléné du sorbitan) dilué à 10 % : 0,3 ml
puis le volume est ajusté à 60 ml avec de l'eau.

Cette suspension aqueuse est ensuite diluée par de l'eau pour obtenir la concentration désirée en matière active.

Des plants de radis (variété Pernot), semés sur un substrat tourbe terre - pouzzolane 50/50 et cultivés en serre, sont traités au stade cotylédons par pulvérisation de la suspension de matière active décrite ci-dessus.

Des plants, utilisés comme témoins, sont traités par pulvérisation d'une solution aqueuse ne contenant pas la matière active.

24 heures après traitement, les plants sont contaminés par pulvérisation d'une solution aqueuse de spores (40000 sp/ml) récoltées sur une culture agée de 13 jours.

Après contamination, les plants sont placés à 18-20°C, en atmosphère saturée en humidité. La notation, en comparaison avec les plants témoins, est effectuée 6 jours après contamination.

Dans ces conditions, on observe à la dose de 1 g/l, une bonne protection (au moins 75%) ou totale avec les composés 1, 2, 16, 17, 19, 23 à 28, 30, 31..

Ces résultats montrent clairement les bonnes propriétés fongicides des dérivés selon l'invention contre les maladies fongiques des plantes dûes à des champignons appartenant aux familles les plus diverses telles que les Phycomycètes, en particulier le mildiou de la vigne, les Basidiomycètes, en particulier les rouilles *Puccinia sp.,* les *Alternaria sp.* les Ascomycètes , les Adelomycètes ou fungi imperfecti, en particulier les *Botrytis sp., Pyricularia oryzae.*

Pour leur emploi pratique, les composés selon l'invention sont rarement utilisés seuls. Le plus souvent ces composés font partie de compositions. Ces compositions, utilisables comme agents fongicides, contiennent comme matière active un composé selon l'invention tel que décrit précédemment en mélange avec les supports solides ou liquides, acceptables en agriculture et les agents tensio-actifs également acceptables en agriculture. En particulier sont utilisables les supports inertes et usuels et les agents tensio-actifs usuels. Ces compositions font également partie de l'invention.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... Plus généralement les composés utilisés dans l'invention peuvent être combinés à tous les additifs solides ou liquides correspondant aux techniques habituelles de la mise en formulation.

D'une façon générale, les compositions selon l'invention contiennent habituellement de 0,05 à 95 % environ (en poids) d'un composé selon l'invention (appelé par la suite matière active), un ou plusieurs supports solides ou liquides et, éventuellement, un ou plusieurs agents tensioactifs.

Par le terme "support", dans le présent exposé, on désigne une matière organique ou minérale, naturelle ou synthétique, avec laquelle le composé est combiné pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau ; alcools, notamment le butanol etc...).

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents. La présence d'au moins un agent tensioactif est généralement indispensable lorsque le composé et/ou le support inerte ne sont pas solubles dans l'eau et que l'agent vecteur de l'application est l'eau.

Ainsi donc, les compositions à usage agricole selon l'invention peuvent contenir les matières actives selon l'invention dans de très larges limites, allant de 0,05 % à 95 % (en poids). Leur teneur en agent tensio-actif est avantageusement comprise entre 5 % et 40 % en poids.

Ces compositions selon l'invention sont elles-mêmes sous des formes assez diverses, solides ou liquides.

Comme formes de compositions solides, on peut citer les poudres pour poudrage (à teneur en composé pouvant aller jusqu'à 100 %) et les granulés, notamment ceux obtenus par extrusion, par compactage, par imprégnation d'un support granulé, par granulation à partir d'une poudre (la teneur en composé dans ces granulés étant entre 0,5 et 80 % pour ces derniers cas), les comprimés ou tablettes effervescents.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes, les gels.

Les concentrés émulsionnables ou solubles comprennent le plus souvent 10 à 80 % de matière active, les émulsions ou solutions prêtes à l'application contenant, quant à elles, 0,001 à 20 % de matière active.

En plus du solvant, les concentrés émulsionnables peuvent contenir quand c'est nécessaire, 2 à 20 % d'additifs appropriés comme les stabilisants, les agents tensio-actifs, les agents de pénétration, les inhibiteurs de corrosion, les colorants ou les adhésifs précédemment cités.

A partir de ces concentrés, on peut obtenir par dilution avec de l'eau des émulsions de toute concentration désirée, qui conviennent particulièrement à l'application sur les cultures.

A titre d'exemple, voici la composition de quelques concentrés émulsionnables :

### Exemple CE 1 :

- matière active 400 g/l
- dodécylbenzène sulfonate alcalin 24 g/l
- nonylphénol oxyéthylé à 10 molécules d'oxyde d'éthylène 16 g/l
- cyclohexanone 200 g/l
- solvant aromatique q.s.p.1 litre

Selon une autre formule de concentré émulsionnable, on utilise :

### Exemple CE 2

- matière active 250 g
- huile végétale époxydée 25 g
- mélange de sulfonate d'alcoylaryle et d'éther de polyglycol et d'alcools gras 100 g
- diméthylformamide 50 g
- xylène 575 g

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0.1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici une composition de suspension concentrée :

### Exemple SC 1 :

- matière active 500 g
- phosphate de tristyrylphénol polyéthoxylé 50 g
- alkylphénol polyéthoxylé 50 g
- polycarboxylate de sodium 20 g
- éthylène glycol 50 g
- huile organopolysiloxanique (antimousse) 1 g
- polysaccharide 1,5 g
- eau 316,5 g

Les poudres mouillables (ou poudre à pulvériser) sont habituellement préparées de manière qu'elles contiennent 20 à 95 % de matière active, et elles contiennent habituellement, en plus du support solide, de 0 à 30 % d'un agent mouillant, de 3 à 20 % d'un agent dispersant, et, quand c'est nécessaire, de 0,1 à 10 % d'un ou plusieurs stabilisants et/ou autres additifs, comme des agents de pénétration, des adhésifs, ou des agents antimottants, colorants, etc...

Pour obtenir les poudres à pulvériser ou poudres mouillables, on mélange intimement les matières actives dans les mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables trés avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

A titre d'exemple, voici diverses compositions de poudres mouillables (ou poudres à pulvériser) :

### Exemple PM 1

- matière active 50%
- alcool gras éthoxylé (agent mouillant) 2,5%
- phényléthylphénol éthoxylé (agent dispersant) 5%
- craie (support inerte) 42,5%

### Exemple PM 2 :

- matière active 10%
- alcool synthétique oxo de type ramifié, en C13 éthoxylé par 8 à 10 oxyde d'éthylène (agent mouillant) 0,75%
- lignosulfonate de calcium neutre (agent dispersant) 12%
- carbonate de calcium (charge inerte) q.s.p. 100 %

### Exemple PM 3 :

Cette poudre mouillable contient les mêmes ingrédients que dans l'exemple précédent, dans les proportions ci-après :
- matière active 75%
- agent mouillant 1,50%
- agent dispersant 8%
- carbonate de calcium (charge inerte) q.s.p. 100%

### Exemple PM 4 :

- matière active 90%
- alcool gras éthoxylé (agent mouillant) 4%
- phényléthylphénol éthoxylé (agent dispersant) 6%

### Exemple PM 5 :

- matière active 50%
- mélange de tensio-actifs anioniques et non ioniques (agent mouillant) 2,5%
- lignosulfonate de sodium (agent dispersant) 5%
- argile kaolinique (support inerte) 42,5%

Les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les composés selon l'invention peuvent être formulés sous la forme de granulés dispersibles dans l'eau également compris dans le cadre de l'invention.

Ces granulés dispersibles, de densité apparente généralement comprise entre environ 0,3 et 0,6 ont une dimension de particules généralement comprise entre environ 150 et 2000 et de préférence entre 300 et 1500 microns.

La teneur en matière active de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale ou, de préférence, organique. On a obtenu d'excellents résultats avec l'urée. Dans le cas d'une charge insoluble, celle-ci est de préférence minérale, comme par exemple le kaolin ou la bentonite. Elle est alors avantageusement accompagnée d'agents tensio-actifs (à raison de 2 à 20 % en poids du granulé) dont plus de la moitié est, par exemple, constituée par au moins un agent dispersant, essentiellement anionique, tel qu'un polynaphtalène sulfonate alcalin ou alcalino terreux ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc...). On termine généralement par un concassage suivi d'un tamisage à la dimension de particule choisie dans les limites mentionnées ci-dessus.

De préférence, il est obtenu par extrusion, en opérant comme indiqué dans les exemples ci-après.

### Exemple GD1 : Granulés dispersibles

Dans un mélangeur, on mélange 90 % en poids de matière active et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

### Exemple GD2 : Granulés dispersibles

Dans un mélangeur, on mélange les constituants suivants :
- matière active 75%
- agent mouillant (alkylnaphtalène sulfonate de sodium) 2%
- agent dispersant (polynaphtalène sulfonate de sodium) 8%
- charge inerte insoluble dans l'eau (kaolin) 15%

Ce mélange est granulé en lit fluide, en présence d'eau, puis séché, concassé et tamisé de manière à obtenir des granulés de dimension comprise entre 0,15 et 0,80 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

En ce qui concerne les compositions adaptées au stockage et au transport, elles contiennent plus avantageusement de 0,5 à 95 % (en poids) de substance active.

L'invention a également pour objet l'utilisation des composés selon l'invention pour la lutte contre les maladies fongiques des plantes par traitement préventif ou curatif, sur le feuillage ou le matériel de propagation, de ces dernières ou de leur lieu de croissance.

## Revendications

1. Dérivés de 3-arylpyrazoles de formule: 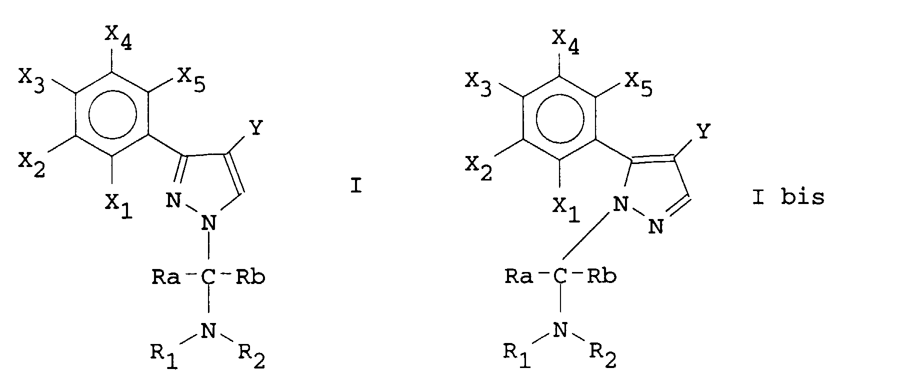 dans laquelle :
X₁, X₂, X₃, X₄ et X₅, identiques ou différents, sont:
- un atome d'hydrogène ou d'halogène, un groupe hydroxy, mercapto, cyano, thiocyanato, nitro, nitroso, amino éventuellement substitué par un ou deux alkyles ou phényles ou acyle, les imines, énamines , amidines et guanidines dérivées de ce groupe amino;
- un radical alkyle, hydroxyalkyle, alkoxyalkyle, alkylthioalkyle, alkylsulfinylalkyl, alkylsulfonylalkyl, benzyle, alkényle, alkynyle, cyanoalkyle, alkoxy, alkenyloxy, formyle, acétyle, alkyl- ou alkoxy(thio)-carbonyle, mono ou dialkylamino(thio)carbonyle, iminocarbonyl, mono ou diarylamino(thio)carbonyle, carboxyle, carboxylate, carbamoyle ou benzoyle,
- un radical phényle, phénoxy, phénylthio,
- un radical alkyle- ou alkoxy- ou mono ou dialkylamino- ou phényl-sulfényl ou sulfinyle ou sulfonyle,
- un groupe phosphoryle, substitué par deux groupes choisis dans le groupe comprenant alkyle, alkoxy, alkylthio, dialkylamino, benzyloxy, phényloxy et phényl,
- un groupe trialkyl- ou alkylphényle -silyle
étant entendu que dans toutes les significations hydrocarbonées ci-dessus, la partie alkyle de ces groupes peut comprendre de 1 à 4 atomes de carbone et être éventuellement halogénée et que phényle désigne le noyau phényle éventuellement substitué.
Deux des X₁, X₂, X₃, X₄ et X₅ adjacents peuvent également former un cycle carboné comprenant au total de 5 à 7 chainons, qui peut comporter un ou plusieurs atomes ou groupes suivants: O, S, N, C=O, C=S, SO, SO₂, CH=CH. Les carbones de ce pont peuvent ou non être substitués par au moins un atome d'halogène et/ ou au moins un groupe hydroxy, alkoxy, alkylthio, mono ou dialkylamino, alkylsulfinyle ou -sulfonyle, la partie alcoyle étant telle que définie ci-dessus,
sous réserve qu'au moins un des groupes X soit différent d'hydrogène;
Y est:
- un atome d'hydrogène ou d'halogène,
- un groupe hydroxy, mercapto, et leurs dérivés formylés, alkyl- ou alcoxy- ou amino (thio)acylés, le groupement amino pouvant être éventuellement substitué.
- un groupe nitro, cyano, thiocyanato, azido
- un groupe alkyle, alkényle, alkynyle, alkoxy ou alkylthio, chacun de ces groupes étant éventuellement halogéné,
- un radical acyle ou thio acyle qui peut être: un radical formyle, un groupe alkyl- ou alkényl-carbonyle ou -thincarbonyle, le radical alkyle ou alkényle pouvant être linéaire ou ramifié,
- un groupe alkoxy- ou alkylthio- ou amino- ou monoalkylamino- ou dialkylamino- ou phénylamino- ou alkylphenylamino(thio)carbonyle
- un groupe carboxy ou ses sels,
- un phénoxy éventuellement substitué,
- un amino substitué ou non par un ou deux alkyles ou phényles,
- un groupe alkylsulfinyle ou alkylsulfonyle, la partie alkyle étant telle que définie ci-dessus,
Y et X₅ pouvant également former un cycle carboné comprenant au total de 5 à 7 chainons, qui peut comporter un ou plusieurs atomes ou groupes suivants: O, S, N, C=O, C=S, SO, SO₂, CH=CH. Les carbones de ce pont pouvant ou non être substitués par au moins un atome d'halogène et/ ou au moins un groupe hydroxy, alkoxy, alkylthio, mono ou dialkylamino, alkylsulfinyle ou -sulfonyle, la partie alcoyle étant telle que définie ci-dessus.
Ra est
- un atome d'hydrogène,
- un radical R pouvant être:
- alkyl de 1 à 6 C(éventuellement substitué par GR7)
- cycloalkyl(3 à7 C), cycloalkyl(3 à7 C)alkyl(1 à 4C)éventuellement substitué par GR3(défini ci-après))
- phényl, un hétérocycle Het(défini ci-après), phénylalkyl(1 à 4C), Het alkyl (1 à 4C), éventuellement substitué par 1 à 8 atomes d'halogène et GR4(défini ci-après),
- un groupe C(V)-V'R₀ ou C(V)NR₀R'₀, dans lesquels:
- V et V', identiques ou différents sont un atome d'oxygène ou de soufre et
- R₀ et R'₀ identiques ou différents peuvent être est un atome d'hydrogène, les radicaux R ou R', éventuellement substitués par un groupe α, qui peut être un groupe GR₁ (défini ci-après), NZ₁Z₂, SO₂NZ₁Z₂, CVZ₁Z₂, dans lesquels V est comme défini plus haut et Z₁,Z₂ sont un atome d'hydrogène ou le radical R, qui peut en outre éventuellement substitué par GR2(défini ci-après),
Rb est un atome d'hydrogène ou peut former avec Ra, un cycle hydrocarboné de 4 à 6 C,
R₁ est:
- 1) R₀, dans lequel Z₁, Z₂ peuvent en outre former. avec l'atome d'azote auquel ils sont rattachés, un radical Het (défini ci-après), éventuellement substitué par GR3(défini ci-après); ou un radical R' pouvant être: alkényl, alkynyl, phénylalkényl, phénylalkynyl, Hetalkényl, Hetalkynyl, la partie alkényl ou alkynyl de ces radicaux ayant de 2 à 6C,
Lorsque R1 est un groupe R0, et plus précisément lorsque R0 est Hetalkyl (1 à 4 C), il peut être plus spécifiquement un groupe de formule IIa ou IIb, 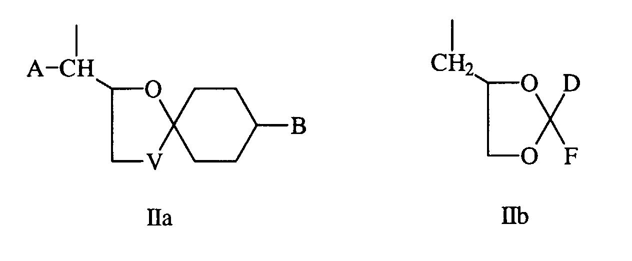 dans lesquels,
V a la même définition que précédemment,
A est un atome d' hydrogène ou un groupe méthyle,
Sous réserve que V est un atome d' oxygène lorsque A est un groupe méthyle.
B est un groupe: alkyl (1 à 14 C), cycloalkyl (3 à 7 C), cycloalkylalkyl (3 à 7 C; 1 à 8 C pour la partie alkyl), aryl (6 à 10 C), aralkyl ( 6 à 10 C; 1 à 8 C pour la partie alkyl). D est un groupe:
Alkyl (1 à 14 C),
Halogénoalkyl (1 à 14 C pour la partie alkyl; 1 à 6 atomes d' halogène),
Cyanalkyl (1 à 4 C pour la partie alkyl),
Aralkyl, Aryloxy(thio)alkyl, Arylsulfi(o)nylalkyl, Aralkyloxy(thio)alkyl (6 à 10 C pour la partie Aryl; 1 à 8 C pour la partie alkyl; la partie Aryl éventuellement substituée par un ou plusieurs substituant(s) choisi(s) parmi le groupe GR4).
F, identique à ou différent de D, et pouvant avoir les mêmes significations, peut être en outre un atome d' hydrogène.
- 2) R₃, qui peut être:
- un atome d'hydrogène,
- un groupe SO₂W₁, dans lequel W₁ est le groupe R ou R'
- un groupe C(V)W₂, dans lequel W₂ est un groupe R₀, VR₀, NR₀R'₀, dans lequel R₀ est tel que défini plus haut,
- un groupe P(V)W₃, W'₃ dans lequel W₃, W'₃, identiques ou différents peuvent être un groupe R, VR ou R', W₃, W'₃, pouvant en outre former, avec l'atome de phosphore auquel ils sont rattachés, un radical Het, éventuellement substitué par GR4(défini ci-après),
- un groupe CW₄=N-W'₄, dans lequel W₄ et W'₄, identiques ou différents peuvent être un groupe nitro, cyano, R₀, VR₀, C(V)V'R₀, et C(V)NR₀R'₀, dans lesquel R₀ et R'₀ sont tels que définis plus haut, R₀ pouvant en outre, pour C(V)V'R₀, être un atome de métal alcalin ou alcalinoterreux; W₄ et W'₄, pouvant en outre former, avec l'atome d'azote auquel ils sont rattachés, un radical Het, éventuellement substitué par GR3(défini ci-après);
- 3) un groupe G1-T1-Rc, dans lequel :
- G1 est un groupe SO₂,C(V), P(V)W3 et C=N-W4,
- T1 est V, CH ou Cα,
G1 et T1 pouvant en outre former un groupe N=CW4, les symboles V, W3, W4 et α étant tels que définis ci-dessus;
- Rc est un alkyle de 1 à 6 C;
- 4) un groupe C(O)-R₀, dans lesquel R₀ est tel que défini plus haut,
- 5) un groupe NTT', dans lequel:
- T est R₀, G1-T1-Rc ou N=CW3W4
et T' est R₀ ou R₃, Rc , G2-T2 Rc ou G1-T1 Rc;
R₀, R₃, Rc, G1, G2, T1 et T2 étant tels que définis ci-dessus;
G2 et T2 étant respectivement identiques à ou différents de G1 et T1; et T et T' ne pouvant être ensemble un atome d'hydrogène et pouvant former un groupe morpholino ou pipéridino;
- 6) un groupe C(CRaRbNR₅)ₙCRaRbR₄, dans lequel n est égal à zéro ou 1, Ra, Rb sont tels que définis plus haut, R₄ est un radical de formule: 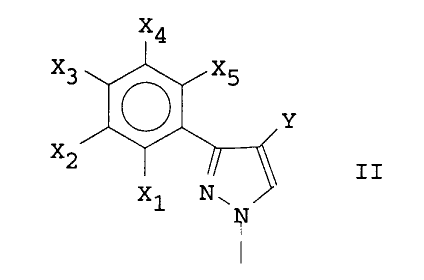 et R₅ est R1 ou CRaRbR₂
- 7) un groupe C(L)NT'T", dans lequel L est V, T" est R₀, G1-T1-Rc ou CRaRbR₄, et dans lequel Ra, Rb, R₄ , V et T' sont tels que définis ci-dessus, T' et T" ne pouvant être à la fois un atome d'hydrogène et pouvant former un groupe morpholino ou pipéridino;
R₂, identique à ou différent de R₁, et pouvant avoir les mêmes significations, c'est à dire pouvant être R₀, OR₀, R₃, Rc, G1-T1-Rc, G2-T2-Rc, et CRaRbR₄, sous réserve qu'ils ne soient pas simultanément un atome d'hydrogène, R₁ et R₂ pouvant en outre former, avec l'atome d'azote auquel ils sont rattachés:
- un radical Het azoté, éventuellement substitué par GR3, de préférence un morpholino ou pipéridino; ou pyrazolidino;
- un groupe N=CH-W5, dans lequel W5 est R, Rb étant un atome d'hydrogène,
et les sels(halogénures,carboxylates et sulfates) d'ammonium substitué et d'imidinium substitué de ces composés;
étant donné que dans ce qui précède, sauf indication spéciale:
"alkyl" désigne un alkyl contenant de 1 à 4 atomes de carbone,
"cycloalkyl" désigne un cycloalkyl contenant de 1 à 4 atomes de carbone,
"alkényl" ou "alkynyl" désigne un alkényl ou alkynyl de 2 à 5 atomes de carbone, "Het" est un radical hétérocyclique,mono ou bicyclique, contenant de 5 à 10 atomes de carbone, dont 1 à 4 sont N, O, S ou P.
GR1 est:
- un atome d'halogène ou un groupe nitro ou cyano,
- un groupe R, R', OR, RS(O)m, avec m égal à 1 à 3, RC(V)V' ou R'C(V)V'
GR2 est:
- un atome d'halogène ou un groupe nitro ou cyano,
- un groupe R, R', OR, RS(O)m, avec m égal à 1 ou 3, RC(V)V' ou R'C(V)V',
- un groupe mono ou dialkyl amino, mono ou dialkyl amino(thio) carbonyl, mono ou dialkyl aminosulfonyl.
GR3 est:
- un atome d'halogène ou un groupe nitro ou cyano,
- un groupe R, OR, RS(O)m, avec m égal à 1 ou 3, RC(V)V' ou
- un groupe mono ou dialkyl amino, mono ou dialkyl amino(thio)carbonyl, mono ou dialkyl aminosulfonyl.
GR4 est:
- un atome d'halogène ou un groupe nitro ou cyano,
- alkyl, alkoxy, alkylsulfényl, alkylsulfonyl, alkyl(thio)carbonyl, alkoxy(thio)carbonyl,
- un groupe mono ou dialkyl amino, mono ou dialkyl amino(thio) carbonyl, mono ou dialkyl aminosulfonyl.
GR5 est :
- un atome d'halogène ou un groupe nitro ou cyano,
- un groupe R, R', OR, RS(O)3, RC(V)V' ou R'C(V)V',
- un groupe mono ou dialkyl amino, mono ou dialkyl amino(thio) carbonyl, mono ou dialkyl aminosulfonyl.
GR6 est GR5 sauf R' et R'C(V)V'.
GR7 est GR2 sauf alkyl;
tous les radicaux de ces substituants étant définis ci-dessus.

2. Composés selon la revendication 1, dans la formule desquels X₁, X₂, X₃, X₄ et X₅ sont choisis dans le groupe comprenant un atome d'hydrogène ou
d'halogène, cyano, nitro, ou un radical alkyle de 1 à 4 atomes de carbone ou deux X voisins forment avec le phényle un benzodioxole éventuellement halogéné.

3. Composés selon la revendication 1, dans la formule desquels Y est un atome d'halogène ou un groupe cyano;

4. Composés selon la revendication 1, dans la formule desquels Ra et Rb, identiques ou différents, sont un atome d'hydrogène ou un alkyle de 1 à 4 atomes de carbone.

5. Composés selon la revendication 1, dans la formule desquels R₁ et R₂, identiques ou différents, sont un radical R.

6. Composés selon la revendication 5, dans la formule desquels R₁ et R₂, identiques ou différents, sont un alkyle de 1 à 4 atomes de carbone.

7. Composés selon la revendication 1, dans la formule desquels R₁ et R₂ forment, avec l'atome d'azote qui les porte un groupe morpholino, pipéridino, pyrolidino, imidazolyl, triazolyl, pyrazolyl ou benzoxazinyl.

8. Procédé de préparation de composés de formule I selon l'une des revendication 1 à 7, dans laquelle R₁ et R₂, identiques ou différents sont choisis dans R₀, caractérisé en ce qu'on fait agir un aldéhyde RaCH(O) ou d'une cétone RaRbC(O), en présence d'une amine R₁R₂NH, sur un phényl pyrazole de formule II-H 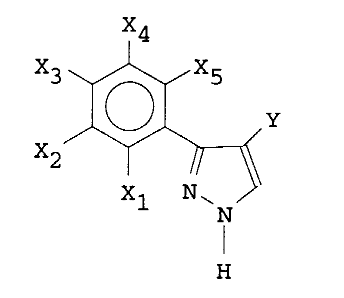 dans laquelle X₁ à X₅ et Y ont les mêmes significations que dans la formule I, à une température comprise entre l'ambiante et le point de reflux, en milieu solvant, éventuellement en présence d'une quantité catalytique d'acide fort.

9. Procédé de préparation de sels d'ammonium substitué ou d'imidinium substitué des composés de formule I selon l'une des revendication 1 à 7, caractérisé en ce qu'on fait réagir un pyrazole de formule R₄CRaRbU, U est un atome d'halogène ou un groupe libérable tel que mésytyle ou tolyle, sur une quantité stoechiométrique d'une amine tertiaire RNR₁R₂, aliphatique ou hétérocyclique à au moins un azote salifiable, à une température comprise entre l'ambiante et le point de reflux, en milieu solvant, ou un mélange de solvants, ou un solvant ou un mélange de solvants, aprotique choisi dans le groupe comprenant l'acétone, l'acétonitrile ou encore un solvant, ou un mélange de solvants, protique polaire choisi dans le groupe comprenant le méthanol, l'éthanol, ou encore polaire tel que le N,N-diméthylformamide et la N-méthyl pyrrolidone.

10. Procédé de préparation de composés de formule I selon l'une des revendication 1 à 7, dans laquelle R₁ₑₜ R₂ sont un groupe CRaRbR₄, c'est à dire de formule R₄CRaRbNCRaRbR₄, caractérisé en ce qu'on fait réagir un hydroxyméthyl-pyrazole de formule R₄CRaRbOH sur un pyrazole dimère de formule R₄CRaRbNHCRaRbR₄ ou sur du formamide, en milieu solvant aromatique ou protique polaire, à une température comprise entre l'ambiante et le point de reflux, en milieu solvant, éventuellement en présence d'une quantité catalytique d'acide fort.

11. Procédé de préparation de composés de formule I selon l'une des revendication 1 à 7, dans laquelle R₁ et R₂ forment ensemble avec l'atome d'azote qui les porte, un groupe N=CH-W5, caractérisé en ce qu'on fait agir un aldédyhe RaCH(O), en présence d'ammoniac alcoolique, sur un phényl pyrazole de formule II-H 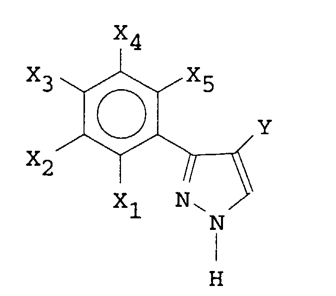 dans laquelle X₁, X₂, X₃, X₄ et X₅ et Y ont les mêmes significations que dans la formule I, à une température comprise entre l'ambiante et le point de reflux, en milieu solvant protique polaire, éventuellement en présence d'une quantité catalytique d'acide fort.

12. Compositions pour la protection des plantes contre les maladies fongiques, caractérisées en ce qu'elles contiennent comme matière active au moins un composé selon l'une des revendications 1 à 7.

13. Composés de formules R4CRaRbNHCRaRbR4, R4CRaRbOH), R4CRaNHCHO dans lesquelles:
Ra est
- un atome d'hydrogène,
- un radical R pouvant être:
- alkyl de 1 à 6 C(éventuellement substitué par GR7)
- cycloalkyl(3 à7 C), cycloalkyl(3 à7 C)alkyl(1 à 4C)éventuellement substitué par GR3(défini ci-après))
- phényl, un hétérocycle Het(défini ci-après), phénylalkyl(1 à 4C), Het alkyl (1 à 4C), éventuellement substitué par 1 à 8 atomes d'halogène et
GR4(défini ci-après),
- un groupe C(V)-V'R₀ ou C(V)NR₀R'₀, dans lesquels:
- V et V', identiques ou différents sont un atome d'oxygène ou de soufre et
- R₀ et R'₀ identiques ou différents peuvent être est un atome d'hydrogène, les radicaux R ou R', éventuellement substitués par un groupe α, qui peut être un groupe GR₁ (défini ci-après), NZ₁Z₂, SO₂NZ₁Z₂, CVZ₁Z₂, dans lesquels V est comme défini plus haut et Z₁,Z₂ sont un atome d'hydrogène ou le radical R, qui peut en outre éventuellement substitué par GR2(défini ci-après),
Rb est un atome d'hydrogène ou peut former avec Ra, un cycle hydrocarboné de 4 à 6 C, et
R₄ est un radical de formule: 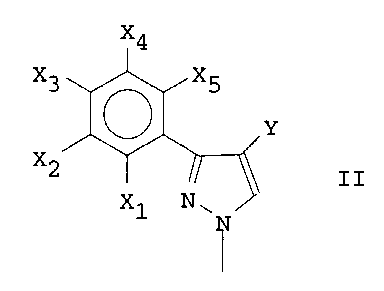 dans laquelle
X₁, X₂, X₃, X₄ et X₅, identiques ou différents, sont:
- un atome d'hydrogène ou d'halogène, un groupe hydroxy, mercapto, cyano, thiocyanato, nitro, nitroso, amino éventuellement substitué par un ou deux alkyles ou phényles ou acyle, les imines, énamines , amidines et guanidines dérivées de ce groupe amino;
- un radical alkyle, hydroxyalkyle, alkoxyalkyle, alkylthioalkyle, alkylsulfinylalkyl, alkylsulfonylalkyl, benzyle, alkényle, alkynyle, cyanoalkyle, alkoxy, alkenyloxy, formyle, acétyle, alkyl- ou alkoxy(thio)-carbonyle, mono ou dialkylamino(thio)carbonyle, iminocarbonyl, mono ou diarylamino(thio)carbonyle, carboxyle, carboxylate, carbamoyle ou benzoyle,
- un radical phényle, phénoxy, phénylthio,
- un radical alkyle- ou alkoxy- ou mono ou dialkylamino- ou phényl-sulfényl ou sulfinyle ou sulfonyle,
- un groupe phosphoryle, substitué par deux groupes choisis dans le groupe comprenant alkyle, alkoxy, alkylthio, dialkylamino, benzyloxy, phényloxy et phényl,
- un groupe trialkyl- ou alkylphényle -silyle
étant entendu que dans toutes les significations hydrocarbonées ci-dessus, la partie alkyle de ces groupes peut comprendre de 1 à 4 atomes de carbone et être éventuellement halogénée et que phényle désigne le noyau phényle éventuellement substitué.
Deux des X₁, X₂, X₃, X₄ et X₅ adjacents peuvent également former un cycle carboné comprenant au total de 5 à 7 chainons, qui peut comporter un ou plusieurs atomes ou groupes suivants: O, S, N, C=O, C=S, SO, SO₂, CH=CH. Les carbones de ce pont peuvent ou non être substitués par au moins un atome d'halogène et/ ou au moins un groupe hydroxy, alkoxy, alkylthio, mono ou dialkylamino, alkylsulfinyle ou -sulfonyle, la partie alcoyle étant telle que définie ci-dessus,
sous réserve qu'au moins un des groupes X soit différent d'hydrogène;
utilisables comme intermédiaires.

## Patentansprüche

1. 3-Arylpyrazolderivate der Formel: 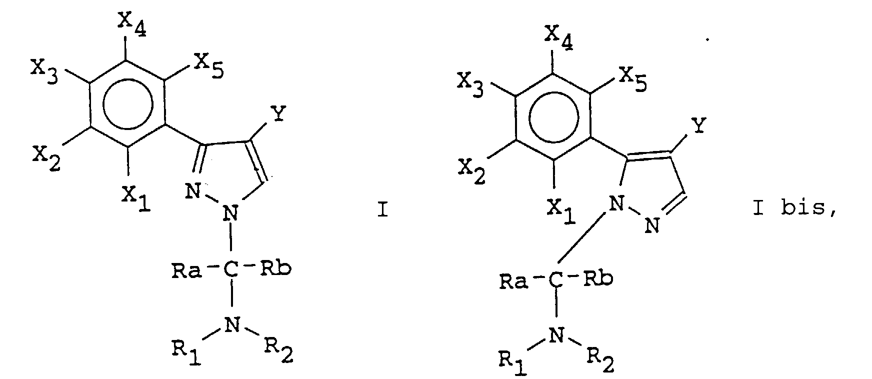 worin bedeuten:
X₁, X₂, X₃, X₄ und X₅, die identisch oder voneinander verschieden sind:
- Wasserstoff oder Halogen, Hydroxy, Mercapto, Cyano, Thiocyanato, Nitro, Nitroso, Amino, das gegebenenfalls mit einer oder zwei Alkyl-, Phenyl- oder Acylgruppen substituiert ist, und die von dieser Aminogruppe abgeleiteten Imine, Enamine, Amidine und Guanidine;
- Alkyl, Hydroxyalkyl, Alkoxyalkyl, Alkylthioalkyl, Alkylsulfinylalkyl, Alkylsulfonylalkyl, Benzyl, Alkenyl, Alkinyl, Cyanoalkyl, Alkoxy, Alkenyloxy, Formyl, Acetyl, Alkyl- oder Alkoxy(thio)carbonyl, Mono- oder Dialkylamino(thio)carbonyl, Iminocarbonyl, Mono- oder Diarylamino(thio)carbonyl, Carboxy, Carboxylat, Carbamoyl oder Benzoyl,
- Phenyl, Phenoxy, Phenylthio,
- Alkyl-, Alkoxy-, Mono- oder Dialkylamino- oder Phenyl-sulfenyl oder -sulfinyl oder -sulfonyl,
- Phosphoryl, das mit zwei Gruppen substituiert ist, die unter Alkyl, Alkoxy, Alkylthio, Dialkylamino, Benzyloxy, Phenyloxy und Phenyl ausgewählt sind,
- Trialkyl- oder Alkylphenyl-silyl,
mit der Maßgabe, daß in allen oben genannten Kohlenwasserstoffgruppen der Alkylteil dieser Gruppen 1 bis 4 Kohlenstoffatome aufweisen und gegebenenfalls Halogen enthalten kann, und daß Phenyl den gegebenenfalls substituierten Phenylring bezeichnet.
Zwei aneinander grenzende Gruppen X₁, X₂, X₃, X₄ und X₅ können ferner einen fünf- bis siebengliedrigen Kohlenstoffring bilden, der ein oder mehrere der folgenden Gruppen enthalten kann: O, S, N, C=O, C=S, SO, SO₂, CH=CH. Die enthaltenen Kohlenstoffatome können gegebenenfalls mit mindestens einem Halogenatom und/oder mindestens einer Gruppe Hydroxy, Alkoxy, Alkylthio, Mono - oder Dialkylamino, Alkylsulfinyl oder -sulfonyl substituiert sein, wobei der Alkylteil die oben angegebene Bedeutung aufweist;
mit der Maßgabe, daß mindestens eine der Gruppen X von Wasserstoff verschieden ist;
Y:
- Wasserstoff oder Halogen,
- Hydroxy, Mercapto und deren Formylderivate und Alkyl-, Alkoxy- oder Amino(thio)acylderivate, wobei die Aminogruppe gegebenenfalls substituiert sein kann,
- Nitro, Cyano, Thiocyanato, Azido,
- Alkyl, Alkenyl, Alkinyl, Alkoxy oder Alkylthio, wobei jede der Gruppen gegebenenfalls halogeniert ist,
- Acyl oder Thioacyl, das sein kann: Formyl, Alkyl- oder Alkenylcarbonyl oder Alkyl- oder Alkenylthiocarbonyl, wobei die Gruppen Alkyl oder Alkenyl geradkettig oder verzweigt sein können,
- Alkoxy-, Alkylthio-, Amino-, Monoalkylamino-, Dialkylamino, Phenylamino- oder Alkylphenylamino(thio)carbonyl,
- Carboxy oder seine Salze,
- Phenoxy, das gegebenenfalls substituiert ist,
- Amino, das gegebenenfalls mit einer oder zwei Alkyl- oder Phenylgruppen substituiert ist,
- Alkylsulfinyl oder Alkysulfonyl, wobei der Alkylteil die oben definierte Bedeutung aufweist,
wobei Y und X₅ einen fünf- bis siebengliedrigen Kohlenstoffring bilden können, der eine oder mehrere der folgenden Atome oder Gruppen enthalten kann: O, S, N, C=O, C=S, SO, SO₂, CH=CH. Die enthaltenen Kohlenstoffatome können gegebenenfalls mit einem Halogenatom und/oder mindestens einer Gruppe Hydroxy, Alkoxy, Alkythio, Mono- oder Dialkylamino, Alkylsulfinyl oder -sulfonyl substituiert sein, wobei der Alkylteil die oben definierte Bedeutung aufweist.
Ra:
- Wasserstoff,
- eine Gruppe R, die bedeuten kann:
- C₁₋₆-Alkyl, das gegebenenfalls mit GR7 substituiert ist,
- C₃₋₇-Cycloalkyl, C₃₋₇-Cycloalkyl-C₁₋₄-Alkyl, gegebenenfalls mit GR3 (im nachfolgenden definiert) substituiert,
- Phenyl, einen Heterocyclus Het (im nachfolgenden definiert), C₁₋₄-Phenylalkyl, Het-C₁₋₄-Alkyl, gegebenenfalls mit 1 bis 8 Halogenatomen und GR4 (im nachfolgenden definiert) substituiert,
- eine Gruppe C(V)-V'R₀ oder C(V)NR₀R'₀, worin bedeuten:
- V und V', die identisch oder voneinander verschieden sind, ein Sauerstoff- oder Schwefelatom und
- R₀ und R'₀, die identisch oder voneinander verschieden sind, Wasserstoff, die Gruppen R und R', die gegebenenfalls mit einer Gruppe α substituiert sind, die eine Gruppe GR1 (im nachfolgenden definiert), NZ₁Z₂, SO₂NZ₁Z₂, CVZ₁Z₂ bedeuten kann, wobei V die oben angegebene Bedeutung aufweist und Z₁ und Z₂ Wasserstoff oder die Gruppe R bedeuten, die ferner gegebenenfalls mit GR2 (im nachfolgenden definiert) substituiert sein kann,
Rb bedeutet Wasserstoff oder Rb kann mit Ra einen C₄₋₆-Kohlenwasserstoffring bilden,
R₁:
1) R₀, worin Z₁ und Z₂ ferner mit dem Stickstoffatom, an das sie gebunden sind, eine Gruppe Het (im nachfolgenden definiert), bilden können gegebenenfalls substituiert mit GR3 (im nachfolgenden definiert); oder eine Gruppe R', die bedeuten kann: Alkenyl, Alkinyl, Phenylalkenyl, Phenylalkinyl, Hetalkenyl, Hetalkinyl, wobei der Alkenyl- oder Alkinylteil dieser Gruppen 2 bis 6 Kohlenstoffatome aufweist,
falls R₁ eine Gruppe R₀ ist und genauer, falls R₀ Hetalkyl (1 bis 4 C) bedeutet, kann R₁ genauer eine Gruppe der Formel IIa oder IIb sein: 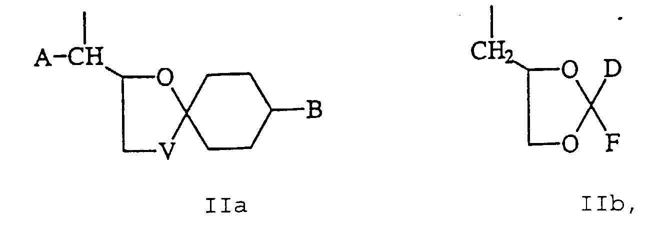 worin:
V die oben angegebene Bedeutung aufweist, A Wasserstoff oder Methyl bedeutet, mit der Maßgabe, daß V ein Sauerstoffatom ist, wenn A Methyl bedeutet.
B eine Gruppe C₁₋₁₄-Alkyl, C₃₋₇-Cycloalkyl, C₃₋₇-Cycloalkyl-C₁₋₈-Alkyl, C₆₋₁₀-Aryl, C₆₋₁₀-Aralkyl bedeutet, wobei die Alkylgruppe 1 bis 8 Kohlenstoffatome aufweist.
D eine Gruppe C₁₋₁₄-Alkyl, Halogenalkyl (1bis 14 C am Alkylteil; 1 bis 6 Halogenatome), C₁₋₄-Cyanoalkyl, Aralkyl, Aryloxy(thio)alkyl, Arylsulfi(o)nylalkyl, Aralkyloxy(thio)alkyl (Aryl: 6 bis 10 C; Alkyl: 1 bis 8 C; Aryl gegebenenfalls substituiert mit einem oder mehreren Substituenten, die unter GR4 ausgewählt sind) bedeutet.
F, das mit D identisch oder davon verschieden ist und die für D angegebenen Bedeutungen aufweisen kann, kann ferner Wasserstoff bedeuten.
2) R₃, das bedeuten kann:
- Wasserstoff,
- SO₂W₁, wobei W₁ die Gruppe R oder R' bedeutet,
- eine Gruppe C(V)W₂, worin W₂ eine Gruppe R₀, VR₀, NR₀R'₀ ist, wobei R₀ die oben angegebene Bedeutung aufweist,
- eine Gruppe P(V)W₃W'₃, wobei W₃ und W'₃, die identisch oder voneinander verschieden sind, eine Gruppe R, VR oder R' bedeuten können, oder W₃ und W'₃ können ferner mit dem Phosphoratom, an das sie gebunden sind, eine Gruppe Het bilden, die gegebenenfalls mit GR4 (im nachfolgenden definiert) substituiert ist,
- ein Gruppe CW₄=N-W'₄, wobei W₄ und W'₄, die identisch oder voneinander verschieden sind, eine Gruppe Nitro, Cyano, R₀, VR₀, C(V)V'R₀ und C(V)NR₀R'₀ sein können, worin R₀ und R'₀ die oben angegebenen Bedeutungen haben und R₀ ferner im Falle von C(V)V'R₀ ein Alkali- oder Erdalkaliatom bedeuten kann, und wobei W₄ und W'₄ ferner mit dem Stickstoffatom, an das sie gebunden sind, eine gegebenenfalls mit GR3 (im nachfolgenden definiert) substituierte Gruppe Het bilden können;
3) eine Gruppe G1-T1-Rc, worin bedeuten:
- G1 eine Gruppe SO₂, C(V), P(V)W₃ und C=N-W₄,
- T₁ eine Gruppe V, CH oder Cα,
wobei G₁ und T₁ ferner eine Gruppe N=CW₄ bilden können, wobei V, W₃, W₄ und α die oben angegebenen Bedeutungen aufweisen,
- eine Rc C₁₋₆-Alkylgruppe;
4) eine Gruppe C(O)-R₀, wobei R₀ die oben angegebene Bedeutung aufweist,
5) eine Gruppe NTT', worin bedeuten:
- T R₀, G₁-T₁-Rc oder N=CW₃W₄ und
- T' R₀ oder R₃, Rc, G₂-T₂Rc oder G₁-T₁Rc, wobei R₀, R₃, Rc, G₁, G₂, T₁ und T₂ die oben angegebenen Bedeutungen aufweisen, G₂ und T₂ identisch zu G₁ und T1 oder davon verschieden sind und T und T' nicht gleichzeitig ein Wasserstoffatom bedeuten können und gemeinsam eine Gruppe Morpholino oder Piperidino bilden können;
6) eine Gruppe C(CRaRbNR₅)ₙCRaRbR₄, wobei n Null oder 1 ist und Ra und Rb die oben angegebenen Bedeutungen aufweisen, R₄ eine Gruppe der Formel 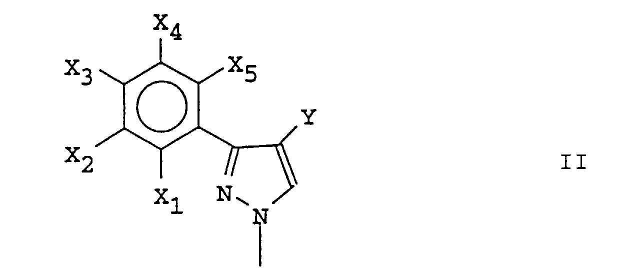 ist und R₅ R₁ oder CRaRbR₂ bedeutet,
7) eine Gruppe C(L)NT'T", wobei L V bedeutet und T" R₀, G1-T1-Rc oder CRaRbR₄ ist, wobei Ra, Rb, R₄, V und T' die oben angegebenen Bedeutungen aufweisen, T' und T" nicht gleichzeitig Wasserstoff bedeuten können und gemeinsam eine Gruppe Morpholino oder Piperidino bilden können;
R₂, das identisch zu R₁ oder davon verschieden ist, kann die gleichen Bedeutungen wie R₁ aufweisen, d.h., R₂ kann R₀, OR₀, R₃, Rc, Gl-T1-Rc und CRaRbR₄ bedeuten, mit der Maßgabe, daß die beiden Gruppen nicht gleichzeitig Wasserstoff bedeuten, wobei R₁ und R₂ ferner mit dem Stickstoffatom, an das sie gebunden sind, bilden können
- eine Gruppe Stickstoff-Het, die gegebenenfalls mit GR3 substituiert ist, vorzugsweise eine Gruppe Morpholino, Piperidino oder Pyralzolidino,
- eine Gruppe N=CWH-W5, worin W5 R bedeutet, wobei Rb ein Wasserstoffatom ist, und substituierte Ammonium- und Imidiniumsalze (Halogenide, Carboxylate und Sulfate) dieser Verbindungen,
mit der Maßgabe, daß, falls nicht spezielles angegeben ist,
"Alkyl" eine Alkylgruppe mit 1 bis 4 Kohlenstoffatomen,
"Cycloalkyl" eine Cycloalkylgruppe mit 1 bis 4 Kohlenstoffatomen,
"Alkenyl" oder "Alkinyl" eine Alkenyl- oder Alkinylgruppe mit 2 bis 5 Kohlenstoffatomen und Het eine heterocyclische mono- oder bicyclische Gruppe mit 5 bis 10 Kohlenstoffatomen, wobei von diesen 1 bis 4 N, O, S oder P sind.
GR1 ist:
- Halogen, Nitro oder Cyano,
- eine Gruppe R, R', OR, RS(O)m, wobei m eine Zahl von 1 bis 3 bedeutet, RC(V)V' oder R'C(V)V'.
GR2 ist:
- Halogen, Nitro oder Cyano,
- eine Gruppe R, R', OR, RS(O)m, wobei m eine Zahl 1 oder 3 ist, RC(V)V' oder R'C(V)V'
- Mono- oder Dialkylamino, Mono- oder Dialkylamino(thio)carbonyl, Mono- oder Dialkylaminosulfonyl.
GR3 ist:
- Halogen, Nitro oder Cyano,
- R, OR, RS(O)m, wobei m 1 oder 3 bedeutet, RC(V)V' oder
- Mono- oder Dialkylamino, Mono- oder Dialkylamino(thio)carbonyl oder Mono- oder Dialkylaminosulfonyl.
GR4 ist:
- Halogen, Nitro oder Cyano,
- Alkyl, Alkoxy, Alkylsulfenyl, Alkylsulfonyl, Alkyl(thio)carbonyl, Alkoxy(thio)carbonyl,
- Mono- oder Dialkylamino, Mono- oder Dialkyl. amino(thio)carbonyl oder Mono- oder Dialkylaminosulfonyl.
GR5 ist:
- Halogen, Nitro oder Cyano,
- eine Gruppe R, R', OR, RS(O)3, RC(V)V' oder R'C(V)V',
- Mono- oder Dialkylamino, Mono- oder Dialkylamino(thio)carbonyl, Mono- oder Dialkylaminosulfonyl.
GR6 ist GR5 außer R' und R'C(V)V'.
GR7 ist GR2 außer Alkyl;
wobei alle Gruppen dieser Substituenten die oben angegebenen Bedeutungen aufweisen.

2. Verbindungen nach Anspruch 1, wobei X₁, X₂, X₃, X₄ und X₅ unter Wasserstoff, Halogen, Cyano, Nitro oder Alkyl mit 1 bis 4 Kohlenstoffatomen ausgewählt sind, oder zwei benachbarte X bilden mit Phenyl ein gegebenenfalls halogeniertes Benzodioxol.

3. Verbindungen nach Anspruch 1, wobei Y Halogen oder Cyano bedeutet.

4. Verbindungen nach Anspruch 1, wobei Ra und Rb, die gleich oder voneinander verschieden sind, Wasserstoff oder Alkyl mit 1 bis 4 Kohlenstoffatomen bedeuten.

5. Verbindungen nach Anspruch 1, wobei R₁ und R₂, die identisch oder voneinander verschieden sind, eine Gruppe R bedeuten.

6. Verbindungen nach Anspruch 5, wobei R₁ und R₂, die identisch oder voneinander verschieden sind, Alkyl mit 1 bis 4 Kohlenstoffatomen bedeuten.

7. Verbindungen nach Anspruch 1, wobei R₁ und R₂ mit dem Stickstoffatom, an das sie gebunden sind, eine Gruppe Morpholino, Piperidino, Pyrolidino, Imidazolyl, Triazolyl, Pyrazolyl oder Benzoxazinyl bilden.

8. Verfahren zur Herstellung von Verbindungen der Formel I nach einem der Ansprüche 1 bis 7, wobei R₁ und R₂, die identisch oder voneinander verschieden sind, unter R₀ ausgewählt sind, dadurch gekennzeichnet, daß ein Aldehyl RaCH(O) oder ein Keton RaRbC(O) in Gegenwart eines Amins R₁R₂NH mit einem Phenylpyrazol der Formel II-H 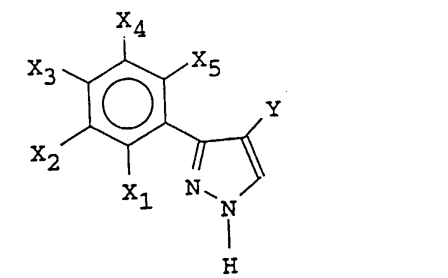 worin X₁ bis X₅ und Y die für Formel I angegebenen Bedeutungen aufweisen, bei einer Temperatur im Bereich von Raumtemperatur bis zur Rückflußtemperatur in einem Lösungsmittelmedium gegebenenfalls in Gegenwart einer katalytischen Menge einer starken Säure umgesetzt wird.

9. Verfahren zur Herstellung der substituierten Ammonium- oder Imidiniumsalze der Verbindungen der Formel I nach einem der Ansprüche 1 bis 7, dadurch gekennzeichnet, daß ein Pyrazol der Formel R₄CRaRbU, wobei U Halogen oder eine freisetzbare Gruppe wie Mesityl oder Tolyl bedeutet, mit einer stöchiometrischen Menge eines tertiären Amins RNR₁R₂, das aliphatisch oder heterocyclisch ist und mindestens ein Stickstoffatom aufweist, das ein Salz bilden kann, bei einer Temperatur im Bereich von Raumtemperatur bis zur Rückflußtemperatur in einem Lösungsmittelmedium oder einem Gemisch von Lösungsmitteln oder einem aprotischen Lösungsmittel oder Gemisch von Lösungsmitteln, das unter Aceton und Acetonitril ausgewählt ist, oder auch einem polaren protischen Lösungsmittel oder Gemisch von Lösungsmitteln, das unter Methanol und Ethanol ausgewählt ist, oder auch polarem Lösungsmittel, wie N,N-Dimethylformamid und N-Methyl-pyrrolidon umgesetzt wird.

10. Verfahren zur Herstellung der Verbindungen der Formel I nach einem der Ansprüche 1 bis 7, worin R₁ und R₂ eine Gruppe CRaRbR₄ bedeuten, d.h. der Formel R₄CRaRbNCRaRbR₄, dadurch gekennzeichnet, daß ein Hydroxymethylpyrazol der Formel R₄CRaRbOH mit einem Pyrazoldimer der Formel R₄CRaRbNHCRaRbR₄ oder Formamid in einem aromatischen oder polaren protischen Lösungsmittelmedium bei einer Temperatur im Bereich von Raumtemperatur bis zur Rückflußtemperatur gegebenenfalls in Gegenwart einer katalytischen Menge einer starken Säure umgesetzt wird.

11. Verfahren zur Herstellung der Verbindungen der Formel I nach einem der Ansprüche 1 bis 7, worin R₁ und R₂ gemeinsam mit dem Stickstoffatom, an das sie gebunden sind, eine Gruppe N=CH-W5 bilden, dadurch gekennzeichnet, daß ein Aldehyd RaCH(O) in Gegenwart einer alkoholischen Ammoniaklösung mit einem Phenylpyrazol der Formel II-H 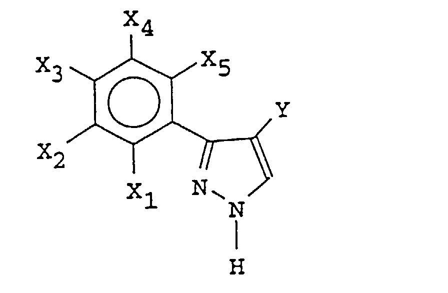 worin X₁, X₂, X₃, X₄, X₅ und Y die in Formel I angegebenen Bedeutungen aufweisen, bei einer Temperatur im Bereich von Raumtemperatur bis zur Rückflußtemperatur in einem protischen polaren Lösungsgmittel gegebenenfalls in Gegenwart einer katalytischen Menge einer starken Säure umgesetzt wird.

12. Zusammensetzungen zum Schutz von Pflanzen gegen Pilzerkrankungen, dadurch gekennzeichnet, daß sie als Wirkstoff mindestens eine Verbindung nach einem der Ansprüche 1 bis 7 enthalten.

13. Verbindungen der Formeln R4CRaRbNHCRaRbR4, R4CRaRbOH, R4CRaNHCHO, worin bedeuten:
Ra
- Wasserstoff,
- R, das bedeuten kann:
- C₁₋₆-Alkyl (gegebenenfalls mit GR7 substituiert),
- C₃₋₇-Cycloalkyl, C₃₋₇-Cycloalkyl-C₁₋₄-Alkyl, gegebenenfalls substituiert mit GR3 (im nachfolgenden definiert),
- Phenyl, einen Heterocyclus Het (im nachfolgenden definiert), C₁₋₄-Phenylalkyl, Het-C₁₋₄-Alkyl, gegebenenfalls mit 1 bis 8 Halogenatomen und GR4 (im nachfolgenden definiert), substituiert
- eine Gruppe C(V)-V'R₀ oder C(V)NR₀R'₀, worin bedeuten:
- V und V', die identisch oder voneinander verschieden sind, ein Sauerstoff- oder Schwefelatom und
- R₀ und R'₀, die identisch oder voneinander verschieden sind, Wasserstoff, die Gruppen R und R', die gegebenenfalls mit einer Gruppe α substituiert sind, die eine Gruppe GR1 (im nachfolgenden definiert), NZ₁Z₂, SO₂NZ₁Z₂, CVZ₁Z₂ bedeuten kann, worin V die oben angegebene Bedeutung hat und Z₁ und Z₂ Wasserstoff oder die Gruppe R bedeuten, die ferner gegebenenfalls mit GR2 (im nachfolgenden definiert) substituiert sein kann,
Rb Wasserstoff oder Rb bildet mit Ra einen Kohlenwasserstoffring mit 4 bis 6 Kohlenstoffatomen und
R₄ ein Gruppe der Formel 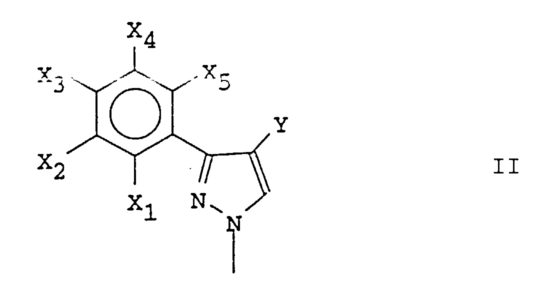 worin X₁, X₂, X₃, X₄ und X₅, die gleich oder voneinander verschieden sind, bedeuten:
- Wasserstoff oder Halogen, Hydroxy, Mercapto, Cyano, Thiocyanato, Nitro, Nitroso, Amino, das gegebenenfalls mit einer oder zwei Alkyl-, Phenyl- oder Acylgruppen substituiert ist, und die von dieser Aminogruppe abgeleiteten Imine, Enamine, Amidine und Guanidine;
- Alkyl, Hydroxyalkyl, Alkoxyalkyl, Alkylthioalkyl, Alkylsulfinylalkyl, Alkylsulfonylalkyl, Benzyl, Alkenyl, Alkinyl, Cyanoalkyl, Alkoxy, Alkenyloxy, Formyl, Acetyl, Alkyl- oder Alkoxy(thio)carbonyl, Mono- oder Dialkylamino(thio)carbonyl, Iminocarbonyl, Mono- oder Diarylamino(thio)carbonyl, Carboxy, Carboxylat, Carbamoyl oder Benzoyl,
- Phenyl, Phenoxy, Phenylthio,
- Alkyl-, Alkoxy-, Mono- oder Dialkylamino- oder Phenyl-sulfenyl oder -sulfinyl oder -sulfonyl,
- Phosphoryl, das mit zwei Gruppen substituiert ist, die unter Alkyl, Alkoxy, Alkylthio, Dialkylamino, Benzyloxy, Phenyloxy und Phenyl ausgewählt sind,
- Trialkyl- oder Alkylphenyl-silyl,
mit der Maßgabe, daß in allen oben genannten Kohlenwasserstoffgruppen der Alkylteil dieser Gruppen 1 bis 4 Kohlenstoffatome aufweisen und gegebenenfalls Halogen enthalten kann, und daß Phenyl den gegebenenfalls substituierten Phenylring bezeichnet.
Zwei aneinander grenzende Gruppen X₁, X₂, X₃, X₄ und X₅ können ferner einen fünf- bis siebengliedrigen Kohlenstoffring bilden, der ein oder mehrere der folgenden Gruppen enthalten kann: O, S, N, C=O, C=S, SO, SO₂, CH=CH. Die enthaltenen Kohlenstoffatome können gegebenenfalls mit mindestens einem Halogenatom und/oder mindestens einer Gruppe Hydroxy, Alkoxy, Alkylthio, Mono- oder Dialkylamino, Alkylsulfinyl oder -sulfonyl substituiert, wobei der Alkylteil die oben angegebene Bedeutung aufweist,
mit der Maßgabe, daß mindestens eine der Gruppen X von Wasserstoff verschieden ist,
die als Zwischenprodukte verwendbar sind.

## Claims

1. 3-Arylpyrazole derivatives of formula: 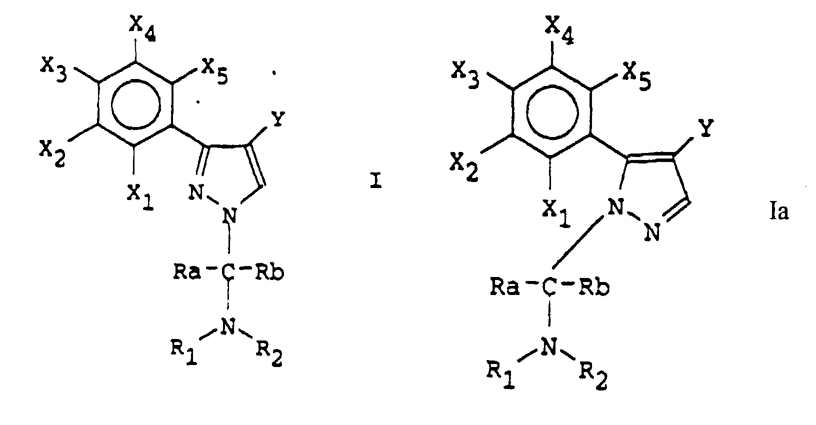 in which:
X₁, X₂, X₃, X₄ and X₅, which may be identical or different, are:
- a hydrogen or halogen atom, a hydroxyl, mercapto, cyano, thiocyanato, nitro or nitroso group, an amino group optionally substituted with one or two alkyl or phenyl or acyl groups, imines, enamines, amidines and guanidines derived from this amino group;
- an alkyl, hydroxyalkyl, alkoxyalkyl, alkylthioalkyl, alkylsulphinylalkyl, alkylsulphonylalkyl, benzyl, alkenyl, alkynyl, cyanoalkyl, alkoxy, alkenyloxy, formyl, acetyl, alkyl- or alkoxy(thio)carbonyl, mono- or dialkylamino(thio)carbonyl, iminocarbonyl, mono- or diarylamino(thio)carbonyl, carboxyl, carboxylate, carbamoyl or -benzoyl radical,
- a phenyl, phenoxy or phenylthio radical,
- an alkyl- or alkoxy- or mono- or dialkylamino- or phenyl-sulphenyl or -sulphinyl or -sulphonyl radical,
- a phosphoryl group substituted with two groups chosen from the group comprising alkyl, alkoxy, alkylthio, dialkylamino, benzyloxy, phenyloxy and phenyl,
- a trialkyl- or alkylphenylsilyl group, it being understood that in all the above hydrocarbon meanings, the alkyl part of these groups may contain from 1 to 4 carbon atoms and may be optionally halogenated and that phenyl denotes the optionally substituted phenyl ring.
Two of the adjacent X₁, X₂, X₃, X₄ and X₅ groups may also form a carbon ring containing 5 to 7 members in total, which may include one or more of the following atoms or groups: O, S, N, C=O, C=S, SO, SO₂, CH=CH. The carbons of this bridge may be unsubstituted or substituted with at least one halogen atom and/or at least one hydroxyl, alkoxy, alkylthio, mono- or dialkylamino, alkyl-sulphinyl or -sulphonyl group, the alkyl part being as defined above, with the proviso that at least one of the X groups is other than hydrogen;
Y is:
- a hydrogen or halogen atom,
- a hydroxyl or mercapto group and their formylated or alkyl- or alkoxy- or amino(thio)acylated derivatives, it being possible for the amino group to be optionally substituted,
- a nitro, cyano, thiocyanato or azido group,
- an alkyl, alkenyl, alkynyl, alkoxy or alkylthio group, each of these groups being optionally halogenated,
- an acyl or thioacyl radical which may be: a formyl radical, an alkyl- or alkenyl-carbonyl or -thiocarbonyl group, it being possible for the alkyl or alkenyl radical to be linear or branched,
- - an alkoxy- or alkylthio- or amino- or monoalkylamino- or dialkylamino- or phenylamino- or alkylphenylamino-(thio)-carbonyl group,
- a carboxyl group or its salts,
- an optionally substituted phenoxy,
- an amino unsubstituted or substituted with one or two alkyls or phenyls,
- an alkylsulphinyl or alkylsulphonyl group, the alkyl part being as defined above,
it also being possible for Y and X₅ to form a carbon ring containing 5 to 7 members in total, which may include one or more of the following atoms or groups: O, S, N, C=O, C=S, SO, SO₂, CH=CH. The carbons of this bridge may be unsubstituted or substituted with at least one halogen atom and/or at least one hydroxyl, alkoxy, alkylthio, mono- or dialkylamino, alkylsulphinyl or -sulphonyl group, the alkyl part being as defined above;
Ra is
- a hydrogen atom,
- a radical R which may be:
- 1 to 6 C alkyl (optionally substituted with GR7)
- (3 to 7 C)cycloalkyl, (3 to 7 C)cycloalkyl(1 to 4 C)alkyl optionally substituted with GR3 (defined below)
- phenyl, a heterocycle Het (defined below), phenyl(1 to 4 C)alkyl, Het(1 to 4 C)alkyl, optionally substituted with 1 to 8 halogen atoms and GR4 (defined below),
- a group C(V)-V'R₀ or C(V)NR₀R'₀, in which:
- V and V', which may be identical or different, are an oxygen or sulphur atom, and
- R₀ and R'₀, which may be identical or different, may be a hydrogen atom, the radicals R and R', optionally substituted with a group α which may be a group GR₁ (defined below), NZ₁Z₂, SO₂NZ₁Z₂, CVZ₁Z₂, in which V is as defined above and Z₁ and Z₂ are a hydrogen atom or the radical R, which may in addition be optionally - substituted with GR2 (defined below),
Rb is a hydrogen atom or may form with Ra a 4 to 6 C hydrocarbon ring,
R₁ is:
- 1) R₀, in which Z₁ and Z₂ may in addition form, with the nitrogen atom to which they are attached, a radical Het (defined below), optionally substituted with GR3 (defined below); or a radical R' which may be: alkenyl, alkynyl, phenylalkenyl, phenylalkynyl, Hetalkenyl or Hetalkynyl, the alkenyl or alkynyl part of these radicals having from 2 to 6 C,
When R1 is a group R0, and more precisely when R0 is Het(1 to 4 C)alkyl, it may more specifically be a group of formula IIa or IIb, 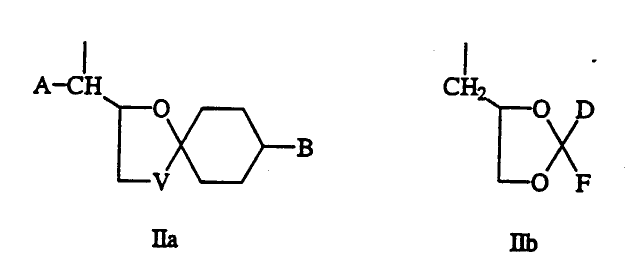 in which,
V has the same definition as above,
A is a hydrogen atom or a methyl group,
with the proviso that V is an oxygen atom when A is a methyl group.
B is a group: (1 to 14 C)alkyl, (3 to 7 C)cycloalkyl, cycloalkylalkyl (3 to 7 C; 1 to 8 C for the alkyl part), (6 to 10 C)aryl, aralkyl (6 to 10 C; 1 to 8 C for the alkyl part).
D is a group:
(1 to 14 C)alkyl, haloalkyl (1 to 14 C for the alkyl part; 1 to 6 halogen atoms),
- cyanalkyl (1 to 4 C for the alkyl part), aralkyl, aryloxy(thio)alkyl, arylsulphi(o)nylalkyl, aralkyloxy(thio)alkyl (6 to 10 C for the aryl part; 1 to 8 C for the alkyl part; the aryl part optionally substituted with one or more substituent(s) chosen from the group GR4).
F, which may be identical to or different from D, and which may have the same meanings, may additionally be a hydrogen atom.
- 2) R₃, which may be:
- a hydrogen atom,
- a group SO₂W₁, in which W₁ is the group R or R',
- a group C(V)W₂, in which W₂ is a group R₀, VR₀ or NR₀R'₀, in which R₀ is as defined above,
- a group P(V)W3, W'3 in which W3, W'3, which may be identical or different, may be a group R, VR or R', it being possible in addition for W3, W'3 to form, with the phosphorus atom to which they are attached, a radical Het, optionally substituted with GR4 (defined below),
- a group CW₄=N-W'₄, in which W₄ and W'₄, which may be identical or different, may be a nitro, cyano, R₀, VR₀, C(V)V'R₀ or C(V)NR₀R'₀ group, in which R₀ and R'₀ are as defined above, it being possible in addition for R₀, for C(V)V'R₀, to be an alkali metal or alkaline-earth metal atom; it being possible in addition for W₄ and W'₄ to form, with the nitrogen atom to which they are attached, a radical Het, optionally substituted with GR3 (defined below),
- 3) a group G1-T1-Rc, in which:
- G1 is a group SO₂, C(V), P(V)W3 or C=N-W4,
- T1 is V, CH or Cα,
it being possible in addition for G1 and
T1 to form a group N=CW4, the symbols V,
W3, W4 and α being as defined above;
- Rc is a 1 to 6 C alkyl;
- 4) a group C(O)-R₀, in which R₀ is as defined above,
- 5) a group NTT', in which:
- T is R₀, G1-T1-Rc or N=CW3W4 and T' is R₀ or R₃, Rc, G2-T2 Rc or G1-T1 Rc;
R₀, R₃, Rc, G1, G2, T1 and T2 being as defined above;
G2 and T2 being respectively identical to or different from G1 and T1; and it not being possible for T and T' together to be a hydrogen atom and it being possible for them to form a morpholino or piperidino group;
- 6) a group C(CRaRbNR₅)ₙCRaRbR₄, in which n is equal to zero or 1, Ra and Rb are as defined above, R₄ is a radical of formula: 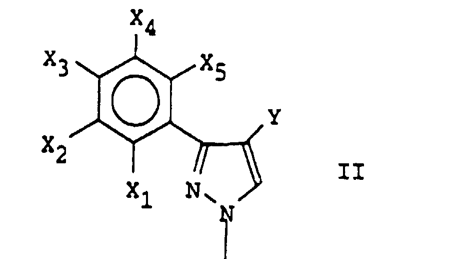 and R₅ is R1 or CRaRbR₂,
- 7) a group C(L)NT'T", in which L is V, T" is R₀, G1-T1-Rc or CRaRbR₄, and in which Ra, Rb, R₄, V and T' are as defined above, it not being possible for T' and T" simultaneously to be a hydrogen atom and it being possible for them to form a morpholino or piperidino group;
R₂, which may be identical to or different from R₁, and which may have the same meanings, that is to say which may be R₀, OR₀, R₃, Rc, G1-T1-Rc, G2-T2-Rc and CRaRbR₄, with the proviso that they are not simultaneously a hydrogen atom,
it being possible in addition for R₁ and R₂ to form, with the nitrogen atom to which they are attached:
- a nitrogen-containing radical Het, optionally substituted with GR3, preferably a morpholino or piperidino; or pyrazolidino;
- a group N=CH-W5, in which W5 is R, Rb being a hydrogen atom,
and the substituted ammonium salts (halides, carboxylates and sulphates) and substituted imidinium salts of these compounds;
given that, in the preceding text, except where especially mentioned:
"alkyl" denotes an alkyl containing from 1 to 4 carbon atoms,
"cycloalkyl" denotes a cycloalkyl containing from 1 to 4 carbon atoms,
"alkenyl" or "alkynyl" denotes an alkenyl or alkynyl having from 2 to 5 carbon atoms,
"Het" is a mono- or bicyclic heterocyclic radical containing from 5 to 10 carbon atoms, of which 1 to 4 are N, O, S or P,
GR1 is:
- a halogen atom or a nitro or cyano group,
- a group R, R', OR, RS(O)m, with m equal to 1 to 3, RC(V)V' or R'C(V)V',
GR2 is:
- a halogen atom or a nitro or cyano group,
- a group R, R', OR, RS(O)m, with m equal to 1 or 3, RC(V)V' or R'C(V)V',
- a mono- or dialkylamino, mono- or dialkylamino(thio)carbonyl, mono- or dialkylaminosulphonyl group,
GR3 is:
- a halogen atom or a nitro or cyano group,
- a group R, OR, RS(O)m, with m equal to 1 or 3, RC(V)V' or
- a mono- or dialkylamino, mono- or dialkylamino(thio)carbonyl, mono- or dialkylaminosulphonyl group,
GR4 is:
- a halogen atom or a nitro or cyano group,
- alkyl, alkoxy, alkylsulphenyl, alkylsulphonyl, alkyl(thio)carbonyl, alkoxy(thio)carbonyl,
- a mono- or dialkylamino, mono- or dialkylamino(thio)carbonyl, mono- or dialkylaminosulphonyl group,
GR5 is:
- a halogen atom or a nitro or cyano group,
- a group R, R", OR, RS(O)3, RC(V)V' or R'C(V)V',
- a mono- or dialkylamino, mono- or dialkylamino(thio)carbonyl, mono- or dialkylaminosulphonyl group,
GR6 is GR5 except for R' and R'C(V)V',
GR7 is GR2 except for alkyl;
all the radicals of these substituents being defined above.

2. Compounds according to claim 1, in the formula for which X₁, X₂, X₃, X₄ and X₅ are chosen from the group comprising a hydrogen or halogen atom, cyano, nitro or an alkyl radical having from 1 to 4 carbon atoms or two neighbouring X groups form, with the phenyl, an optionally halogenated benzodioxole.

3. Compounds according to claim 1, in the formula for which Y is a halogen atom or a cyano group.

4. Compounds according to claim 1, in the formula for which Ra and Rb, which may be identical or different, are a hydrogen atom or an alkyl having from 1 to 4 carbon atoms.

5. Compounds according to claim 1, in the formula for which R₁ and R₂, which may be identical or different, are a radical R.

6. Compounds according to claim 5, in the formula for which R₁ and R₂, which may be identical or different, are an alkyl having from 1 to 4 carbon atoms.

7. Compounds according to claim 1, in the formula for which R₁ and R₂ form, with the nitrogen atom which bears them, a morpholino, piperidino, pyrrolidino, imidazolyl, triazolyl, pyrazolyl or benzoxazinyl group.

8. Process for the preparation of compounds of formula I according to one of claims 1 to 7, in which R₁ and R₂, which may be identical or different, are chosen from R₀, characterized in that an aldehyde RaCH(O) or of a ketone RaRbC(O) is reacted, in the presence of an amine R₁R₂NH, with a phenylpyrazole of formula II-H 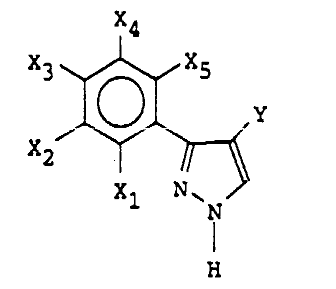 in which X₁ to X₅ and Y have the same meanings as in the formula I, at a temperature between room temperature and the reflux point, in a solvent medium, optionally in the presence of a catalytic amount of strong acid.

9. Process for the preparation of substituted ammonium salts or substituted imidinium salts of the compounds of formula I according to one of claims 1 to 7, characterized in that a pyrazole of formula R₄CRaRbU, U is a halogen atom or a group which may be released such as mesityl or tolyl, is reacted with a stoichiometric amount of an aliphatic or heterocyclic tertiary amine RNR₁R₂ containing at least one nitrogen which may be salified, at a temperature between room temperature and the reflux point, in a solvent medium or mixture of solvents, or an aprotic solvent or mixture of solvents chosen from the group comprising acetone and acetonitrile, or alternatively in a polar protic solvent or mixture of solvents chosen from the group comprising methanol and ethanol, or alternatively a polar solvent or mixture of solvents such as N,N-dimethylformamide and N-methylpyrrolidone.

10. Process for the preparation of compounds of formula I according to one of claims 1 to 7, in which R₁ and R₂ are a group CRaRbR₄, that is to say of formula R₄CRaRbNCRaRbR₄, characterized in that a hydroxymethyl pyrazole of formula R₄CRaRbOH is reacted with a dimeric pyrazole of formula R₄CRaRbNHCRaRbR₄ or with formamide, in an aromatic or polar protic solvent medium, at a temperature between room temperature and the reflux point, in a solvent medium, optionally in the presence of a catalytic amount of strong acid.

11. Process for the preparation of compounds of formula I according to one of claims 1 to 7, in which R₁ and R₂ form, together with the nitrogen atom which bears them, a group N=CH-W5, characterized in that an aldehyde RaCH(O) is reacted, in the presence of alcoholic ammonia solution, with a phenylpyrazole of formula II-H 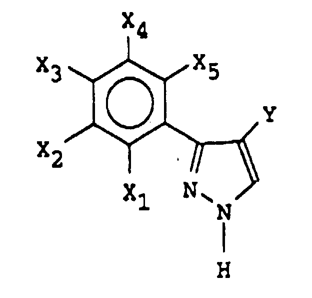 in which X₁, X₂, X₃, X₄ and X₅ and Y have the same meanings as in the formula I, at a temperature between room temperature and the reflux point, in a polar protic solvent medium, optionally in the presence of a catalytic amount of strong acid.

12. Compositions for the protection of plants against fungal diseases, characterized in that they contain as active material at least one compound according to one of claims 1 to 7.

13. Compounds of formulae R4CRaRbNHCRaRbR4, R4CRaRbOH) and R4CRaNHCHO in which:
Ra is
- a hydrogen atom,
- a radical R which may be:
- 1 to 6 C alkyl (optionally substituted with GR7)
- (3 to 7 C)cycloalkyl, (3 to 7 C)cycloalkyl(1 to 4 C)alkyl optionally substituted with GR3 (defined below)
- phenyl, a heterocycle Het (defined below), phenyl(1 to 4 C)alkyl, Het(1 to 4 C)alkyl, optionally substituted with 1 to 8 halogen atoms and GR4 (defined below),
- a group C(V)-V'R₀ or C(V)NR₀R'₀, in which:
- V and V', which may be identical or different, are an oxygen or sulphur atom, and
- R₀ and R'₀, which may be identical or different, may be a hydrogen atom, the radicals R and R', optionally substituted with a group α which may be a group GR₁ (defined below), NZ₁Z₂, SO₂NZ₁Z₂, CVZ₁Z₂, in which V is as defined above and Z₁ and Z₂ are a hydrogen atom or the radical R, which may in addition be optionally substituted with GR2 (defined below),
Rb is a hydrogen atom or may form with Ra a 4 to 6 C hydrocarbon ring, and R₄ is a radical of formula: 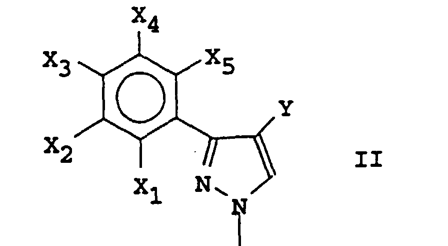 in which
X₁, X₂, X₃, X₄ and X₅, which may be identical or different, are:
- a hydrogen or halogen atom, a hydroxyl, mercapto, cyano, thiocyanato, nitro or nitroso group, an amino group optionally substituted with one or two alkyl or phenyl or acyl groups, imines, enamines, amidines and guanidines derived from this amino group;
- an alkyl, hydroxyalkyl, alkoxyalkyl, alkylthioalkyl, alkylsulphinylalkyl, alkylsulphonylalkyl, benzyl, alkenyl, alkynyl, cyanoalkyl, alkoxy, alkenyloxy, formyl, acetyl, alkyl- or alkoxy(thio)carbonyl, mono- or dialkylamino(thio)carbonyl, iminocarbonyl, mono- or diarylamino(thio)carbonyl, carboxyl, carboxylate, carbamoyl or benzoyl radical,
- a phenyl, phenoxy or phenylthio radical,
- an alkyl- or alkoxy- or mono- or dialkylamino- or phenyl-sulphenyl or -sulphinyl or -sulphonyl radical,
- a phosphoryl group substituted with two groups chosen from the group comprising alkyl, alkoxy, alkylthio, dialkylamino, benzyloxy, phenyloxy and phenyl,
- a trialkyl- or alkylphenylsilyl group,
it being understood that in all the above hydrocarbon meanings, the alkyl part of these groups may contain from 1 to 4 carbon atoms and may be optionally halogenated and that phenyl denotes the optionally substituted phenyl ring.
Two of the adjacent X₁, X₂, X₃, X₄ and X₅ groups may also form a carbon ring containing 5 to 7 members in total, which may include one or more of the following atoms or groups: O, S, N, C=O, C=S, SO, SO₂, CH=CH. The carbons of this bridge may be unsubstituted or substituted with at least one halogen atom and/or at least one hydroxyl, alkoxy, alkylthio, mono- or dialkylamino, alkyl-sulphinyl or -sulphonyl group, the alkyl part being as defined above,
with the proviso that at least one of the X groups is other than hydrogen;
which can be used as intermediates.
